# EUROPÄISCHE PATENTSCHRIFT

(11) **EP 3 718 584 B1**
(45) Veröffentlichungstag und Bekanntmachung des Hinweises auf die Patenterteilung: **17.07.2024**
(21) Anmeldenummer: 20165802.8
(22) Anmeldetag: 26.03.2020
(51) Int. Cl.: A61M 5/34

(54) **VERFAHREN ZUM VERBINDEN EINES ERSTEN BAUTEILS MIT EINEM ZWEITEN BAUTEIL**
METHOD FOR CONNECTING A FIRST COMPONENT TO A SECOND COMPONENT
PROCÉDÉ POUR CONNECTER UN PREMIER COMPOSANT À UN SECOND COMPOSANT

(30) Priorität: 02.04.2019 DE 102019108583
(43) Veröffentlichungstag der Anmeldung: 07.10.2020
(73) Patentinhaber: Gerresheimer Regensburg GmbH, 93047 Regensburg (DE)
(72) Erfinder: DANIEL, Christian, 92533 Wernberg-Köblitz (DE)
(74) Vertreter: Hannke Bittner & Partner mbB Regensburg

(56) Entgegenhaltungen:
- DE-A1- 102010 045 095
- GB-A- 1 215 435

## Beschreibung

Die Erfindung betrifft ein Verfahren zum Verbinden eines ersten Bauteils insbesondere eines Stechmittels mit einem zweiten Bauteil insbesondere einem Spritzenkörper mittels Kleben.

DE 10 2010 045095 beschreibt ein Verfahren dazu, gemäß dem Stand der Technik.

Oftmals ist es erwünscht, dass Spritzen bereits mit einem Stechmittel, beispielsweise einer Nadel oder einer Kanüle versehen ausgeliefert werden. Insbesondere vorgefüllte Spritzen werden bevorzugt mit einem Stechmittel versehen ausgeliefert. Die vorfüllbaren Spritzen(-körper), welche aus Glas oder aus Kunststoff hergestellt sein können, werden von dem Primärpackmittelhersteller unter Reinraumbedingungen hergestellt, abgepackt, sterilisiert und abfüllfertig direkt in den Reinraum des Abfüllers geliefert. Sie können dann ohne weitere Behandlungsschritte zur Abfüllung eingesetzt werden. Die vorfüllbaren Spritzen werden durch den Abfüller von der proximalen Seite, bzw. der Flanschseite befüllt.

Das Stechmittel wird üblicherweise mit einem Klebemittel oder Klebstoff in der Spritze beziehungsweise in einem Stechmittelkanal in dem distalen Endabschnitt der Spritze befestigt. Ein Stechmittel kann hierbei eine Nadel eine Kanüle oder ein ähnliches Mittel sein. Die Schwierigkeit besteht darin, die entsprechenden Kontaktflächen des Stechmittels und des Spritzenkörpers mit dem Klebemittel ausreichend zu benetzen. Um dies zu gewährleisten, wird der Stechmittelkanal mit einem größeren Durchmesser ausgestaltet. Ein derart bemessener Stechmittelkanal erfordert jedoch auch eine größere Menge an Klebemittel, um diesen auszufüllen. Dies zieht einen finanziellen Mehraufwand nach sich. Ein Stechmittelkanal mit einem größeren Durchmesser birgt auch die Gefahr, dass das Klebemittel in den Vorratsbehälter für das Medium gelangt. Bei einigen Medikamenten oder Impfstoffen kann der Kontakt zu dem Klebemittel zu ungewollten Wechselwirkungen führen, welche den Stoff unbrauchbar machen können. Ferner kann ein zu großer Durchmesser des Stechmittelkanals dazu führen, dass die Haltekräfte des Klebers nicht ausreichend sind. Die vorgegebenen Anforderungen für entsprechende Abziehkräfte können somit nicht eingehalten werden.

Auch die Verwendung von sehr niedrig viskosen Klebstoffen ist nachteilig, da diese zur Tröpfchenbildung neigen, wodurch die Handhabung dieser Klebstoffe sich schwierig gestaltet. Ferner besteht auch hier die Gefahr, dass der Klebstoff den Stechmittelkanal durchläuft und in den Vorratsbehälter gelangt.

Die eingangs genannten Probleme hinsichtlich einer Verklebung eines Stechmittels in einem Spritzenkörper lassen sich auch auf ähnlich gelagerte Anordnungen beziehungsweise Verklebungen von anderweitigen ersten Bauteilen in entsprechenden Aufnahmevolumina zweiter Bauteil übertragen.

Aufgabe der vorliegenden Erfindung ist es demnach ein Verfahren bereitzustellen, mittels welchem die oben genannten Nachteile überwunden werden können.

Diese Aufgabe wird gelöst von einem Verfahren gemäß unabhängigem Anspruch 1. Die Abhängigen Ansprüche 2-11 beschreiben weitere, vorteilhafte Ausgestaltungen.

Die nachfolgend in der Beschreibung genannten Schritte a) bis c) sind in Anspruch 1 definiert.

Unter dem Begriff Klebemittel oder auch Klebstoff versteht man einen nichtmetallischen Werkstoff, der Bauteile durch Adhäsion (Oberflächenhaftung) und Kohäsion (innere Festigkeit) miteinander verbindet. Der Querschnitt einer Klebung lässt sich vorteilhafterweise in eine Adhäsionszone und eine Kohäsionszone unterteilen. In der Adhäsionszone verändert das Klebemittel durch die Haftung an der Kontaktoberfläche seine chemische Struktur und Zusammensetzung. Dadurch verändern sich auch die makroskopischen Eigenschaften des Klebemittels. In der Kohäsionszone liegt das Klebemittel in seinem unveränderten Zustand vor. Gemäß der Erfindung ist das Klebemittel ein Urethanmethacrylat.

Üblicherweise ist die Viskosität von Klebemitteln abhängig von der Temperatur. Erfindungsgemäß hat demnach das Klebemittel eine Temperatur T2, bei welcher das Klebemittel auch die benötigte Viskosität aufweist. Diese benötigte Viskosität ist vorzugsweise derart ausgelegt, dass die entsprechende Kontaktoberfläche ausreichend benetzt ist. Andererseits ist die benötigte Viskosität nicht derart niedrig d. h. das Klebemittel derart fließfähig, dass an der Kontaktfläche angrenzende Bereiche mit dem Klebemittel in unerwünschter Weise in Kontakt kommen. Durch das erfindungsgemäße Temperieren des Klebemittels kann demnach die Viskosität des Klebemittels gesteuert werden. Somit kann die Viskosität des Klebemittels durch Erwärmen des Klebemittels erniedrigt werden.

Das erfindungsgemäße Verfahren hat weiterhin den Vorteil, dass zumindest die Kontaktoberflächen des ersten und zweiten Bauteils vor dem Aufbringen des Klebemittels temperiert werden, so dass die Temperatur T1 der ersten und/oder der zweiten Kontaktoberfläche sich von der Temperatur T2 des Klebemittels lediglich durch eine Toleranzabweichung unterscheidet.

Unterscheiden sich die Temperaturen T1 und T2 durch mehr als die Toleranzabweichung voneinander, erfolgt bei einem Kontakt des Klebemittels mit der/den Kontaktoberfläche(en) ein nicht zu vernachlässigender Temperaturausgleich, welcher eine nicht zu vernachlässigende Änderung der Viskosität des Klebemittels zur Folge hat. Durch eine Temperierung der ersten und/oder zweiten Kontaktfläche auf eine Temperatur T1, welche innerhalb der Toleranzabweichung mit der Temperatur T2 übereinstimmt, wird demnach eine nicht vernachlässigbare Temperaturänderung des Klebemittels verhindert. Somit wird auch eine nicht vernachlässigbare Änderung der Viskosität verhindert. Die benötigte Viskosität des Klebemittels kann somit zuverlässig durch eine Temperierung des Klebemittels eingestellt werden und eine Beeinträchtigung der Viskosität durch die Unterschiedliche Temperatur der Kontaktoberflächen wird ausgeschlossen.

Das Verfahren umfasst einen Temperierschritt, mittels welchem das Klebemittel auf die Temperatur T2 gebracht wird.

Alternativ, aber nicht gemäß der Erfindung, wäre es möglich, dass ein bestimmter Klebemittelvorrat permanent auf der Temperatur T2 gehalten wird. Bei einer laufenden Herstellung mehrerer Produkte wird dann vorzugsweise eine vorbestimmte Menge an Klebemittel für jedes einzelne Produkt abgeführt. Diese Menge an Klebemittel hat demnach die Temperatur T2 und somit die gewünscht Viskosität. Ein expliziter Temperierschritt für das Klebemittel wäre somit nicht mehr notwendig. Vorteilhafterweise könnte vor Schritt c), also dem Aufbringen des Klebemittels noch eine Kontrollmessung der Temperatur T2 mittels einer Temperaturmesseinrichtung durchgeführt werden. Eine solche Temperaturmesseinrichtung kann beispielsweise eine kontaktlose Temperaturmessung durchführen. Derartige Temperaturmesseinrichtungen sind beispielsweise Lasertemperaturmessgeräte oder Infrarottemperaturmessgeräte. Alternativ kann die Temperaturmesseinrichtung einen Temperatursensor aufweisen, welcher in oder in unmittelbarer Nähe des Klebemittels angeordnet ist. Die Temperaturmesseinrichtung misst die Temperatur TM und gibt diesen Wert an eine bevorzugte Steuereinrichtung weiter. Diese bevorzugte Steuereinrichtung vergleicht die gemessen Temperatur TM mit der vorgegebenen Temperatur T2. Bei einer Abweichung, welche außerhalb eines vorgegebene Toleranzwerts liegt, wird durch die Steuereinrichtung eine entsprechende Klebemitteltemperiereinrichtung angesteuert, wodurch die Temperatur T2 geregelt wird.

Unter einer Kontaktoberfläche sollen diejenigen Oberflächen verstanden werden, welche mit dem Klebemittel in Kontakt treten sollen, beziehungsweise diejenigen Oberflächen, auf welche das Klebemittel aufgebracht werden soll. Gemäß Schritt c) kann das Klebemittel zunächst auf zumindest einen Abschnitt der ersten Kontaktoberfläche und/oder auf zumindest einen Abschnitt der zweiten Kontaktoberfläche aufgebracht werden.

Bei einem abschnittsweisen Auftrag des Klebemittels kann es vorteilhaft sein, wenn durch einen weiteren Schritt das Klebemittel auf die gesamte erste oder zweite Kontaktoberfläche verteilt wird. Eine solche Verteilung des Klebemittels kann auf verschiedenste Arten erfolgen, beispielsweise durch mechanische Mittel, durch Positionsänderung eines Bauteils oder beider Bauteile relativ zueinander oder durch eine Volumenänderung eines Aufnahmevolumens, in dem das Klebemittel sich befindet. Ferner kann das Klebemittel dadurch auf der Kontaktfläche verteilt werden, dass dieses beispielsweise aufgrund einer Kraft, beispielsweise der Gravitationskraft entlang der Kontaktoberfläche fließt.

Vorteilhafterweise kann in Schritt c) das Klebemittel auf die gesamte erste Kontaktoberfläche und/oder die gesamte zweite Kontaktoberfläche aufgebracht werden.

Eine optimale Klebeverbindung zwischen dem ersten und dem zweiten Bauteil besteht dann, wenn die erste und die zweite Kontaktoberfläche vollständig mit dem Klebemittel bedeckt ist. Bei einer solchen optimalen Klebeverbindung kann die Klebeverbindung den vorgegebenen Auszugs- oder Abzugskräften, zwischen den beiden Bauteilen standhalten.

Nach einer bevorzugten Ausführungsform ist die Temperierung in Schritt b) eine Erwärmung. Nach einer weiteren bevorzugten Ausführungsform ist die Temperierung in Schritt b) eine Abkühlung.

Erfindungsgemäß wird in Schritt b) zumindest die erste und/oder zumindest die zweite Kontaktoberfläche temperiert, so dass diese Kontaktoberflächen eine Temperatur T1 aufweisen, welche innerhalb der Toleranzabweichung der Temperatur T2 entsprechen. Eine solche Temperierung kann vorzugsweise direkt erfolgen oder aber auch indirekt erfolgen. Vorzugsweise trifft bei der direkten Temperierung ein Temperiermedium unmittelbar auf die zumindest eine erste Kontaktoberfläche und/oder die zumindest eine zweite Kontaktoberfläche.

Vorteilhafterweise trifft bei der indirekten Temperierung ein Temperiermedium auf einen Temperierbereich des ersten Bauteils und/oder des zweiten Bauteils, wobei der Temperierbereich des ersten Bauteils und/oder des zweiten Bauteils die Kontaktoberfläche des jeweiligen Bauteils umfasst, umgibt oder in unmittelbarer Nähe zu dieser Kontaktoberfläche angeordnet ist. Vorteilhafterweise trifft bei der indirekten Temperierung ein Temperiermedium auf eine Temperieroberfläche des Temperierbereichs, welche nicht der Kontaktoberfläche entspricht. Vorteilhafterweise erfolgt ein Wärmefluss von der Temperiereoberfläche durch den Temperierbereich zu der entsprechenden Kontaktoberfläche oder ein Wäremefluss von der entsprechenden Kontaktoberfläche zu der Temperieroberfläche. Durch die Richtung des Wärmeflusses wird vorgegeben, ob die entsprechende Kontaktoberfläche erwärmt oder gekühlt wird. Die Kontaktoberfläche kann somit auf die Temperatur T1 gebracht werden. Liegen beispielsweise die entsprechende Kontaktoberfläche innerhalb des ersten oder des zweiten Bauteils, beispielsweise in einem Aufnahmevolumen, könnte die Temperieroberfläche eine äußere Oberfläche des entsprechenden Bauteils sein, welche in unmittelbarer Nähe zu der innenliegenden Kontaktoberfläche ist. Aufgrund einer vorteilhaften Wäremeleitfähigkeit des Materials in dem Temperierbereichs erfolgt nach dem Auftreffen des Temperiermediums und der Temperierung der Temperieroberfläche eine Wärmediffusion zwischen der Kontaktoberfläche und der Temperieroberfläche.

Denkbar wäre es auch, dass das gesamte erste oder zweite Bauteil temperiert wird. Dies ist insbesondere dann vorteilhaft, wenn das entsprechende Bauteil vergleichsweise klein ist. Gemäß einer bevorzugten Ausführungsform wird die Temperatur T1 der ersten Kontaktoberfläche und/oder die Temperatur T1 der zweiten Kontaktoberfläche durch eine Temperaturmesseinrichtung überwacht. Eine solche Temperaturmesseinrichtung kann beispielsweise eine kontaktlose Temperaturmessung durchführen. Derartige Temperaturmesseinrichtungen sind beispielsweise Lasertemperaturmessgeräte oder Infrarottemperaturmessgeräte. Alternativ kann die Temperaturmesseinrichtung einen Temperatursensor aufweisen, welcher an oder in unmittelbarer Nähe der ersten und/oder zweiten Kontaktoberfläche angeordnet ist. Die Temperaturmesseinrichtung misst vorteilhafterweise einen Temperaturwert TM2 der ersten Kontaktoberfläche und/oder der zweiten Kontaktoberfläche. Dieser Temperaturwert wird an eine bevorzugte Steuereinrichtung weitergegeben. Diese bevorzugte Steuereinrichtung vergleicht die gemessene Temperatur TM mit der vorgegebenen Temperatur T2. Bei einer Abweichung, welche außerhalb eines vorgegebene Toleranzwerts liegt, wird durch die Steuereinrichtung eine entsprechende Temperiereinrichtung angesteuert, wodurch die Temperatur T2 geregelt wird.

Gemäß der Erfindung weist das zweite Bauteil ein Aufnahmevolumen auf, welches zumindest abschnittsweise von der zweiten Kontaktoberfläche begrenzt ist. Bevorzugt ist das erste Bauteil abschnittsweise in dem Aufnahmevolumen anordenbar. Vorteilhafterweise liegen bei einer Anordnung des ersten Bauteils in dem Aufnahmevolumen sich die erste und die zweite Kontaktfläche gegenüber. Dabei ist es weiterhin vorteilhaft, wenn bei einer Anordnung des ersten Bauteils in dem Aufnahmevolumen zumindest abschnittsweise zwischen den Kontaktflächen ein Klebehohlraum ausgebildet ist. Vorteilhafterweise ist das erste Bauteil zylindrisch vorzugsweise kreiszylindrisch ausgestaltet. Vorteilhafterweise entspricht die Kontaktoberfläche zumindest abschnittsweise einer äußeren Oberfläche, welche das erste Bauteil bevorzugt vollständig umspannt. Darüber hinaus ist es vorteilhaft, dass das Aufnahmevolumen zylindrisch vorzugsweise kreiszylindrisch ausgestaltet ist. Die Kontaktoberfläche des zweiten Bauteils ist somit vorzugsweise eine innere Oberfläche, welche das Aufnahmevolumen bevorzugt vollständig umgibt, beziehungsweise eingrenzt. Vorteilhafterweise wird das erste Bauteil mittig in das Aufnahmevolumina eingeführt, so dass der Abstand zwischen der ersten und der zweiten Kontaktfläche in Umfangsrichtung konstant ist. Vorteilhafterweise wird der Klebehohlraum in Umfangsrichtung durch die erste und die zweite Kontaktfläche begrenzt. Vorzugsweise wird das Klebemittel in den Klebehohlraum eingeführt, so dass dieser vollständig mit dem Klebemittel ausgefüllt ist. Somit wird vorteilhafterweise eine Klebeverbindung zwischen der ersten und der zweiten Kontaktoberfläche geschaffen.

Nach einer bevorzugten Ausführungsform wird nach Schritt b) das erste Bauteil in das Aufnahmevolumen des zweiten Bauteils zumindest abschnittsweise eingeführt. Dabei ist es vorteilhaft, dass in Schritt c) das Klebemittel auf die gesamte erste Kontaktoberfläche und die gesamte zweite Kontaktoberfläche aufgebracht wird, in dem der Klebeholraum mit Klebemittel ausgefüllt wird. Vorteilhafterweise ist der Klebehohlraum derart orientiert, dass dieser eine obere Öffnung aufweist, welche in vertikaler Richtung über einer unteren Öffnung oder einer unteren Bewandung liegt. Demnach ist es vorteilhaft, dass das Klebemittel durch die obere Öffnung in den Klebehohlraum eingebracht wird. Es wirkt somit vorteilhafterweise unter anderem die Gravitationskraft auf das Klebemittel. Das Klebemittel sinkt demnach nach unten und füllt somit den Klebehohlraum aus. Denkbar wären auch Ausgestaltungen, in welchen alternativ oder kumulativ zur Gravitationskraft eine Kapillarkraft auf das Klebemittel wirkt, wodurch dieses in den Klebehohlraum eindringt. Da durch die Temperierung des Klebemittels auf die Temperatur T2 die Viskosität des Klebemittels auf die Abmessungen des Klebehohlraums abstimmbar ist, kann eine Viskosität eingestellt werden, mittels welcher das Klebemittel in den Klebehohlraum eindringen kann und diesen vollständig ausfüllen kann.

Vorteilhafterweise erfolgt der Schritt c) unmittelbar nach dem Einführen des ersten Bauteils in das Aufnahmevolumen des zweiten Bauteils. Dadurch wird gewährleistet, dass die temperierte erste Kontaktoberfläche und/oder die temperierte zweite Kontaktoberfläche eine Temperatur aufweisen, welche sich noch innerhalb der Toleranzabweichung befindet. Ein eventueller äußerer Temperatureinfluss, also eine eventuelle Abkühlung (oder Erwärmung) der Kontaktoberflächen kann somit vernachlässigt werden.

Nach einer bevorzugten Ausführungsform wird nach Schritt c) das erste Bauteil in das Aufnahmevolumen des zweiten Bauteils zumindest abschnittsweise eingeführt. Vorteilhafterweise wird in Schritt c) das Klebemittel auf zumindest einen Abschnitt der ersten Kontaktoberfläche oder auf zumindest einen Abschnitt der zweiten Kontaktoberfläche aufgebracht. Bevorzugt wird in Schritt c) das Klebemittel auf zumindest einen Abschnitt der zweiten Kontaktoberfläche aufgebracht, in dem das Klebemittel in das Aufnahmevolumen eingebracht wird. Vorteilhafterweise ist die aufgebrachte Menge an Klebemittel derart bemessen, dass der Klebehohlraum ausgefüllt ist. Demnach sind auf die gesamte erste und die gesamte zweite Kontaktoberfläche Klebemittel aufgebracht, nachdem das erste Bauteil in das Aufnahmevolumen des zweiten Bauteils abschnittsweise eingeführt worden ist. Es kann somit eine bestimmte Menge an Klebemittel in (das Aufnahmevolumen) oder auf das erste und/oder zweite Bauteil aufgebracht werden. Durch ein bevorzugtes Zusammenführen des ersten und des zweiten Bauteils wird das Klebemittel auf die gesamten ersten und zweiten Kontaktoberflächen verteilt. Vorzugsweise wird durch die Verringerung des Volumens des Aufnahmevolumens aufgrund des Einführens des ersten Bauteils in das zweite Bauteil das Klebemittel auf die gesamten Kontaktoberflächen verteilt.

Vorteilhafterweise erfolgt das Aufbringen des Klebemittels und das Einführen oder das Zusammenführen des ersten und des zweiten Bauteils innerhalb einer Zeitspanne, innerhalb welcher ein eventueller äußerer Temperatureinfluss, also eine eventuelle Abkühlung (oder Erwärmung) der Kontaktoberflächen vernachlässigt werden kann, so dass die Temperatur T1 weiterhin innerhalb der Toleranzabweichung von T2 liegt.

Gemäß der Erfindung ist die Temperatur T2 größer als die Raumtemperatur. Das Klebemittel wird demnach bevorzugt erwärmt, wodurch die Viskosität des Klebemittels entsprechend gesenkt wird. Typischerweise weisen das erste und das zweite Bauteil vor dem Schritt b) Raumtemperatur, also ca. 20°C auf. Ohne die Temperierung gemäß Schritt b) würde demnach durch den Temperaturausgleich bei Kontakt des Klebemittels mit den Kontaktoberflächen die Klebemitteltemperatur T1 sinken, wodurch die Viskosität des Klebemittels steigt. Eine derartige höhere Viskosität wäre beispielsweise nicht mehr in der Lage, ausreichend weit in den bevorzugten Klebehohlraum einzudringen. Somit wäre der bevorzugte Klebehohlraum nicht ausreichend mit Klebemittel ausgefüllt, wodurch eine unzureichende Klebeverbindung zwischen der ersten und der zweiten Kontaktoberfläche ausgebildet wird. Eine solche unzureichende Klebeverbindung kann nach sich ziehen, dass die Klebeverbindung nur unzureichend niedrigen Auszugskräften, des ersten Bauteils aus dem Aufnahmevolumens des zweiten Bauteils standhält.

Gemäß einer weiteren bevorzugten Ausführungsform weicht die Temperatur T1 von der Temperatur T2 um maximal 5°C ab. Bevorzugt weicht die Temperatur T1 von der Temperatur T2 um maximal 4°C ab. Weiter bevorzugt weicht die Temperatur T1 von der Temperatur T2 um maximal 3°C ab. Weiter bevorzugt weicht die Temperatur T1 von der Temperatur T2 um maximal 1,5°C ab.

Nach einer weiteren bevorzugten Ausführungsform wird die erste Kontaktoberfläche vor Schritt c) vorbehandelt und/oder aktiviert. Vorzugsweise ist diese Vorbehandlung der ersten Kontaktoberfläche ausgewählt aus der Gruppe Reinigung, mechanische Vorbehandlung, chemische Vorbehandlung, thermische Vorbehandlung, physikalische Vorbehandlung. Ferner ist es von Vorteil, dass die zweite Kontaktoberfläche vor Schritt c) vorbehandelt und/oder aktiviert wird. Bevorzugt ist die Vorbehandlung der zweiten Kontaktoberfläche ausgewählt aus der Gruppe Reinigung, mechanische Vorbehandlung, chemische Vorbehandlung, thermische Vorbehandlung, physikalische Vorbehandlung.

Durch eine entsprechende vorteilhafte Vorbehandlung sollen mögliche adhäsionsmindernde Schichten auf den Kontaktoberflächen angetragen werden. Solche Schichten sind auf dem Grundmaterial vorhanden und können beispielsweise Verunreinigungen, absorbierende Medien oder Oxidschichten sein. Derartige Schichten sollten vorteilhafterweise durch eine geeignete Vorbehandlung abgetragen werden. Die Auswahl der Vorbehandlung ist abhängig vom Grundmaterial und den zu erwartenden adhäsionsmindernden Schichten.

Eine bevorzugte Reinigung kann beispielsweise durch Waschen der Kontaktoberflächen beispielsweise mit destilliertem Wasser, Lösemitteln oder Druckluft erfolgen. Eine bevorzugte mechanische Vorbehandlung kann beispielsweise durch Schleifen, Schmirgeln, Bürsten, CO₂-Bestrahlung erfolgen. Eine bevorzugte chemische Vorbehandlung kann beispielsweise durch Beizen mit sauren oder alkalischen Lösungen erfolgen. Eine bevorzugte thermische Vorbehandlung kann beispielsweise durch Beflammung erfolgen. Eine bevorzugte elektrochemische Vorbehandlung kann beispielsweise durch Phosphatierung oder Chromatierung erfolgen. Eine bevorzugte physikalische Vorbehandlung kann beispielsweise durch atmosphärische Plasmabehandlung, Niederdruckplasmaentladung, Corona-Entladung, UV-Behandlung oder Laserbehandlung erfolgen.

Ferner kann es von Vorteil sein, dass die erste und/oder die zweite Kontaktoberfläche vor dem Aufbringen des Klebemittels aktiviert werden. Eine Aktivierung ist eine Modifikation der Kontaktoberfläche, so dass die Benetzung und/oder die Adhäsion des Klebemittels auf/an der Kontaktoberfläche verbessert werden. Insbesondere unpolare Werkstoffe beispielsweise Kunststoffe, die aus langen Polymerketten bestehen (Polypropylen, PE), sind schwer verklebbar, da die Benetzung mit Klebemitteln schwierig ist. Durch eine entsprechende Behandlung wird aus dem unpolaren Werkstoff ein polares Substrat.

Eine solche Aktivierung kann beispielsweise durch eine Plasmabehandlung erfolgen. Die im Plasma vorhandenen reaktiven Spezies erzeugen an den Molekülketten Radikalstellen, an die sich polare Gruppen anlagern können. Wird beispielsweise mit einem Luftplasma gearbeitet, werden in erster Linie sauerstoffhaltige Gruppen, wie Hydroxyl (-OH), Carbonyl (-CO) und Carboxyl (-COOH), in die obersten Moleküllagen eingebunden.

Vorteilhafterweise kann die Aktivierung und die Vorbehandlung, beispielsweise eine Reinigung, der Kontaktoberflächen in einem Arbeitsschritt durch eine Plasmabehandlung erfolgen.

Bei einer vorteilhaften Plasmabehandlung der Kontaktoberflächen wird ein Arbeitsgas verwendet, welches Sauerstoff ist oder enthält. Das Arbeitsgas könnte auch Luft sein. Ferner könnte das Arbeitsgas ein Edelgas beispielsweise Argongas oder ein Gasgemisch sein, welches ein Edelgas enthält. Schließlich könnte das Arbeitsgas ein Formiergas, beispielsweise ein Gas bestehend aus Wasserstoff und Stickstoff sein. Vorzugsweise ist das Plasma ein atmosphärisches Plasma oder ein Niederdruckplasma.

Für die Reinigung der Kontaktoberflächen ist die atmosphärische Plasmabehandlung zur Reinigung besonders gut geeignet, wobei die Effektivität der Plasmabehandlung von der Wahl des Arbeitsgases, dem Ionisationsgrad des Plasmas und der Behandlungsdauer abhängt.

Vorteilhafterweise werden die Bauteile im Zuge eines Niederdruckplasmaverfahren in einer Vakuumkammer platziert. Das Vakuum weist vorzugsweise einen Druck von 10 bis 500 Pa auf.

Nach einer weiteren bevorzugten Ausführungsform werden die erste und/oder die zweite Kontaktoberfläche mit einem Haftvermittler beschichtet. Durch einen Haftvermittler wird eine Art Brücke zwischen dem Klebemittel und der Kontaktoberfläche geschaffen, wodurch die Adhäsion sich verbessert. Vorteilhafterweise besteht der Haftvermittler aus verdünnten Lösungen der Klebemittelrundstoffe. Vorteilhafterweise kann der Haftvermittler durch Walz-, Sprüh- oder Tauchverfahren auf die Kontaktoberflächen aufgebracht werden.

Nach einer bevorzugten Ausführungsform wird zum Temperieren des ersten Bauteils ein erstes Temperiermedium verwendet. Vorteilhafterweise wird zum Temperieren des zweiten Bauteils ein zweites Temperiermedium verwendet. Bevorzugt können das erste und/oder das zweite Temperiermedium ein Fluid, elektromagnetische Strahlung oder ein Plasma sein. Ein Fluid kann hierbei eine Flüssigkeit, beispielsweise Wasser oder ein Gas beispielsweise Luft sein. Eine entsprechende Temperiereinrichtung bringt demnach vorteilhafterweise das Temperiermedium auf die Kontaktoberflächen. Solche Temperiereinrichtungen könnten demnach vorteilhafterweise Heißluftgebläse oder Heizpumpen sein. Ferner kann für den Fall, dass das Temperiermedium elektromagentische Strahlung ist, die Temperiereinrichtung ein Heizstrahler oder Infrarotstrahler sein. Denkbar wäre auch die Verwendung eines Heizdrahtes, der beispielsweise in das bevorzugte Aufnahmevolumen eingeführt wird. Vorzugsweise wird das erste Temperiermedium durch eine erste Temperiereinrichtung dem ersten Bauteil zugeführt. Bevorzugt wird das zweite Temperiermedium durch eine zweite Temperiereinrichtung dem zweiten Bauteil zugeführt.

Enthält das zweite Bauteil ein Aufnahmevolumen, das durch die zweite Kontaktoberfläche begrenzt ist, ist es von Vorteil, wenn das zweite Temperiermedium in das Aufnahmevolumen des zweiten Bauteils eingebracht wird.

Nach einer bevorzugten Ausführungsform wird das zweite Temperiermedium in das Aufnahmevolumen des zweiten Bauteils eingebracht. Die zumindest eine zweite Kontaktoberfläche kann somit entsprechend auf die Temperatur T1 temperiert werden. Eine solche Temperierung ist eine direkte Temperierung. Alternativ kann die zweite Kontaktoberfläche indirekt temperiert werden. Vorteilhafterweise trifft bei der indirekten Temperierung das zweite Temperiermedium auf einen Temperierbereich des zweiten Bauteils, wobei der Temperierbereich des zweiten Bauteils die zweite Kontaktoberfläche umfasst, umgibt oder in unmittelbarer Nähe zu dieser Kontaktoberfläche angeordnet ist. Vorzugsweise ist der Temperierbereich ein Materialabschnitt des zweiten Bauteils welches durch die Temperieroberfläche und zumindest abschnittsweise durch die zweite Kontaktoberfläche begrenzt wird. Vorteilhafterweise trifft bei der indirekten Temperierung ein Temperiermedium auf eine Temperieroberfläche des Temperierbereichs, welche ein Abschnitt einer äußeren Oberfläche des zweiten Bauteils ist. Vorteilhafterweise erfolgt ein Wärmefluss von der Temperieroberfläche durch den Temperierbereich zu der zweiten Kontaktoberfläche, oder von der zweiten Kontaktoberfläche zu der Temperieroberfläche. Je nach Richtung des Wärmeflusses wird die zweite Kontaktoberfläche erwärmt oder abgekühlt. Die zweite Kontaktoberfläche kann somit auf die die Temperatur T1 gebracht werden. Bei einer Erwärmung erfolgt, aufgrund einer vorteilhaften Wärmeleitfähigkeit des Materials in dem Temperierbereich, nach dem Auftreffen des Temperiermediums und der Temperierung der Aufnahmeoberfläche eine Wärmediffusion zu der zweiten Kontaktoberfläche, wodurch diese auf die Temperatur T1 gebracht wird.

Nach einer weiteren Ausführungsform sind die zweite Temperiereinrichtung und eine Aufbringeinrichtung für das Klebemittel in einer Vorrichtung zum Verbinden zweier Bauteile stationär angeordnet. Unter dem Ausdruck stationär ist eine feste Anordnung an einer bestimmten Position zu verstehen. Vorzugsweise wird das zweite Bauteil durch eine erste Transporteinrichtung zu einer Bearbeitungsposition von der zweiten Temperiereinrichtung und/oder von der Aufbringeinrichtung für das Klebemittel geführt. Vorteilhafterweise umfasst die Vorrichtung zum Verbinden zweier Bauteile weiterhin eine zweite Transporteinrichtung, welche das erste Bauteil in das zweite Bauteil einführt oder an das zweite Bauteil anfügt. Vorteilhafterweise führen die erste und zweite Transporteinrichtung eine Vielzahl von ersten beziehungsweise zweiten Bauteilen nacheinander der Bearbeitungsposition, wodurch eine Serienproduktion ermöglicht ist. Die Aufbringeinrichtung für das Klebemittel ist vorteilhafterweise mit einem Klebemittelvorrat verbunden. Das Klebemittel im Klebemittelvorrat wird vorzugsweise mittels einer Klebemitteltemperiereinrichtung temperiert.

Nach einer weiteren vorteilhaften Ausführungsform wird sowohl das erste als auch das zweite Bauteil durch eine einzige Temperiereinrichtung temperiert. Vorteilhafterweise ist das erste Bauteil in einem Aufnahmevolumen des zweiten Bauteils abschnittsweise eingeführt, bevor die Temperierung durchgeführt wird.

Nach einer weiteren bevorzugten Ausführungsform wird das Klebemittel mittels einer Aushärteeinrichtung ausgehärtet. Die Art der Aushärteeinrichtung ist abhängig von der Art des verwendeten Klebemittels. Einige Klebmittel härten beispielsweise bei bestimmten erhöhten Temperaturen aus. Demnach kann die Aushärteeinrichtung bevorzugt eine Wärmequelle sein, beispielsweise ein Infrarotstrahler, ein Heißluftgebläse oder Ähnliches. Andere Klebemittel härten wiederum durch den Einfluss von elektromagnetischer Strahlung wie beispielsweise Licht oder UV-Strahlung. Demnach könnte die Aushärteeinrichtung eine Strahlungsquelle, beispielsweise eine Lampe, eine LED, eine UV-Lampe, eine UV-LED sein. Vorteilhafterweise wird die Strahlungsquelle außerhalb des zweiten Bauteils platziert. Das zweite Bauteil sollte demnach vorteilhafterweise transparent für die entsprechende Strahlung sein. Vorteilhafterweise umfasst die Vorrichtung zum Verbinden zweier Bauteile die Aushärteeinrichtung, wobei vorzugsweise die Bauteile der Aushärteeinrichtung mittels der Transporteinrichtung zugeführt werden.

Weitere Klebemittel insbesondere 2-Komponenten-Klebemittel härten nach der Mischung der beiden Komponenten bei Raumtemperatur aus. Eine entsprechende Aushärteeinrichtung wäre somit nicht notwendig.

Gemäß der Erfindung ist das erste Bauteil ein Stechmittel und das zweite Bauteil ein Spritzenkörper. Vorzugsweise weist der Spritzenkörper einen Vorratsbehälter auf, in welchem ein zur Verabreichung vorgesehenes Medium aufbewahrbar ist. Bevorzugt weist der Vorratsbehälter eine proximale Öffnung des Spritzenkörpers auf. Weiterhin ist es von Vorteil, dass der Spritzenkörper einen distalen Endbereich aufweist, in welchem ein Stechmittelkanal vorgesehen ist. Bevorzugt entspricht der Stechmittelkanal dem Aufnahmevolumen des zweiten Bauteils. Vorteilhafterweise weist der distale Endbereich eine distale Öffnung des Spritzenkörpers auf. Der distale Endbereich kann vorteilhafterweise ein Spritzenkonus sein. Vorteilhafterweise ist der Stechmittelkanal mit dem Vorratsbehälter verbunden. Bevorzugt weist der Spritzenkörper einen Übergangsbereich auf, der zwischen dem distalen Endbereich und dem Vorratsbehälter angeordnet ist. Vorteilhafterweise weist der Vorratsbehälter einen ersten Außendurchmesser und einen ersten Innendurchmesser auf. Bevorzugt weist der distale Endbereich einen zweiten Außendurchmesser und einen zweiten Innendurchmesser auf. Vorteilhafterweise ist der zweiten Außendurchmesser kleiner als der erste Außendurchmesser und der zweite Innendurchmesser kleiner als der erste Innendurchmesser. Vorteilhafterweise geht der erste Innendurchmesser in dem Übergangsbereich in den zweiten Innendurchmesser über. Vorteilhafterweise geht der erste Außendurchmesser in dem Übergangsbereich in den zweiten Außendurchmesser über. Vorteilhafterweise ist der distale Endbereich konisch ausgebildet.

Die Begriffe "distal" und "proximal" sind derart zu verstehen, dass das distale Ende der Spritze näher an dem Applikationsort ist und das proximale Ende weiter weg von dem Applikationsort. Analog sind die Begriffe "distale und proximale Richtung" zu verstehen.

Unter einem Stechmittel sind Nadeln, Kanülen und ähnliche Mittel für derartige Zwecke zu verstehen. Vorteilhafterweise besteht das Stechmittel aus einem Metall, bevorzugt aus Edelstahl.

Nach einer vorteilhaften Ausgestaltung wird das Stechmittel derart in den Stechmittelkanal eingeführt, dass das proximale Ende des Stechmittels nicht über das proximale Ende des Stechmittelkanals in den Übergangsbereich hineinragt. Die erste Kontaktoberfläche erstreckt sich vorteilhafterweise ausgehend vom proximalen Ende des Stechmittels bis zu der Länge des Stechmittels, ab welcher das Stechmittel aus dem Stechmittelkanal hinausragt.

Nach einer weiteren vorteilhaften Ausgestaltung wird das Stechmittel derart in den Stechmittelkanal eingeführt, dass das proximale Ende des Stechmittels über das proximale Ende des Stechmittelkanals in den Übergangsbereich hineinragt. Die Kontaktoberfläche des Stechmittels ist somit zwischen zwei Oberflächen vorgesehen, welche nicht mit Klebemittel benetzt werden.

Nach einer weiteren vorteilhaften Ausführungsform ist es vorteilhaft, wenn der Stechmittelkanal in dem distalen Abschnitt einen größeren Innendurchmesser als in dem proximalen Abschnitt aufweist. In diesem distalen Abschnitt wird das Stechmittel eingesetzt und verklebt. Der Übergangsbereich zu dem proximalen Abschnitt mit dem kleineren Durchmesser kann als Anlagefläche für das Stechmittel dienen. Bevorzugt liegt der Durchmesser des distalen Abschnitts in einem Bereich zwischen 0,7 und 1,3 mm, besonders bevorzugt bei etwa 1 mm. Der Durchmesser des proximalen Abschnitts liegt bevorzugt in einem Bereich zwischen 0,45 mm und 0,9 mm und besonders bevorzugt bei etwa 0,7 mm.

Nach einer weiteren bevorzugten Ausführungsform ist der Spritzenkörper aus Glas gefertigt. Vorzugsweise besteht der Spritzenkörper aus Silikatglas, beispielsweise aus Borosilikatglas oder aus Quarzglas. Derartige Glasspritzen weisen eine hervorragende chemische Resistenz, Neutralität und Undurchlässigkeit auf.

Gemäß einer bevorzugten Ausführungsform besteht der Spritzenkörper aus einem Polymer-Kunststoff, bevorzugt aus einem Polyolefin, beispielsweise Polypropylen oder Polyethylen, besonders bevorzugt aus einem Cyclo-Olefinen Polymer (COP) beziehungsweise aus einem Cyclo-Olefinen Co-Polymer (COC). COC ist im Gegensatz zu den teilkristallinen Polyolefinen, wie Polyethylen und Polypropylen, amorph und damit transparent.

Nach einer vorteilhaften Ausführungsform wird die zweite Kontaktoberfläche indirekt temperiert. Vorzugsweise wird zumindest ein Abschnitt einer äußeren Oberfläche des distalen Endbereichs mittels einer zweiten Temperiereinrichtung temperiert, wobei mittels einer Wärmediffusion von der äußeren Oberfläche des distalen Endbereichs zu der zweiten Kontaktoberfläche oder von der zweiten Kontaktoberfläche zu der äußeren Oberfläche des distalen Endbereichs die zweite Kontaktoberfläche temperiert wird.

Der Abschnitt der äußeren Oberfläche des distalen Endbereichs entspricht somit der Temperieroberfläche. Da der Stechmittelkanal in der Regel kreiszylindrisch ausgestaltet ist, ist die zweite Kontaktoberfläche die innere Oberfläche dieses Hohlzylinders. Vorteilhafterweise ist der distale Endbereich der Temperierbereich. Dabei ist es vorteilhaft, wenn der gesamte distale Endbereich derart temperiert wird, dass diese die Temperatur T1 aufweist.

Dabei wäre es denkbar, dass die zweite Temperiereinrichtung eine Öffnung aufweist, welche in radialer Richtung von dem distalen Endbereich beabstandet ist. Das Temperiermedium tritt aus dieser Öffnung auf und trifft auf einen Abschnitt der äußeren Oberfläche des distalen Endbereichs. Durch die Wärmediffusion in dem Material des distalen Endbereichs wird dieser insgesamt temperiert.

Ferner wäre es möglich, dass die zweite Temperiereinrichtung zumindest zwei Öffnungen aufweist, welche in unterschiedlichen radialen Richtungen von dem distalen Endbereich beabstandet sind. Beispielsweise wäre es möglich zwei solcher Öffnungen diametral gegenüber anzuordnen. Das Temperiermedium kann somit auf zwei sich diametral gegenüberliegenden Abschnitten des distalen Endbereichs auftreffen. Die Temperierung des distalen Endabschnitts durch die Wärmediffusion in dessen Material kann somit schneller erfolgen. Bevorzugt wird durch die die Wärmeleitfähigkeit des Materials des distalen Endbereichs, welches vorteilhafterweise Glas oder ein Polymer-Kunststoff ist die innenliegende zweite Kontaktoberfläche temperiert. Vorteilhafterweise wird die Temperatur des Abschnitts der äußeren Oberfläche durch eine Temperaturmesseinrichtung überwacht. Die Temperatur der zweiten Kontaktoberfläche kann durch die Temperatur der äußeren Oberfläche und mittels weiterer Materialparameter, wie Volumen und Wärmeleitfähigkeit bestimmt werden.

Nach einer weiteren bevorzugten Ausführungsform wird die zweite Kontaktoberfläche direkt temperiert. Vorteilhafterweise wird das zweite Temperiermedium durch eine zweite Temperiereinrichtung in den Stechmittelkanal des distalen Endbereichs eingebracht. Vorzugsweise wird die Temperiereinrichtung an einer distalen Öffnung des Stechmittelkanals platziert. Vorteilhafterweise kann sowohl bei der direkten als auch bei der indirekten Temperierung das zweite Temperiermedium ein Fluid oder elektromagnetische Strahlung sein. Bevorzugte Ausgestaltungen der zweiten Temperiereinrichtung könnten somit beispielsweise Heißluftgebläse, Heizstrahler, Warmwasserpumpen oder Ähnliches sein.

Nach einer weiteren bevorzugten Ausführungsform wird das zweite Temperiermedium durch eine Abführeinrichtung aus dem Spritzenköper entfernt, wobei die Abführeinrichtung in dem Übergangsbereich angeordnet ist. Vorteilhafterweise umfasst die Abführeinrichtung eine Dichtungseinrichtung, welche dichtend an einer Innenwand des Übergangsbereichs angeordnet ist, so dass kein Temperiermedium in den Vorratsbehälter gelangt.

Eine derartige Abführeinrichtung kann beispielsweise eine Absaugeinrichtung sein. Da in dem Vorratsbehälter Medien, beispielsweise Impfstoffe gelagert werden, welche sensibel gegenüber Verunreinigungen reagieren, ist es wünschenswert, dass kein Temperiermedium, beispielsweise Fluid, Plasma, usw. in den Vorratsbehälter gelangt. Dies wird vorteilhafterweise durch die Abführeinrichtung, welche durch die Dichteinrichtung dichtend an einer Innenwand des Übergangsbereichs angeordnet ist, gewährleistet.

Gemäß der Erfindung wird als Klebemittel ein Urethanmethacrylat verwendet. Bei 25°C weist ein solche Klebemittel eine Viskosität von 1000 bis 2000 mPa s auf.

Gemäß der Erfindung liegt die eine ausreichende Erniedrigung der Viskosität um den Klebehohlraum zu füllen, in einem Temperaturbereich zwischen 39°C und 46°C vorzugsweise bei 42°C. Ein solches vorteilhaftes Klebemittel könnte das Produkt LOCITINE^{®} 3345 sein.

Die Aufgabe wird ebenso gelöst von einer Vorrichtung zum Verbinden zweier Bauteile, insbesondere von einer Vorrichtung zum Bestücken eines Spritzenkörpers mit einem Stechmittel.

Bevorzugt ist die Vorrichtung dazu geeignet und bestimmt, die beschriebenen Ausführungsformen des Verfahrens (insbesondere einzelne und/oder mehrere) auszuführen. Vorteilhafterweise umfasst die Vorrichtung eine erste Transporteinrichtung und eine zweite Transporteinrichtung in Form eines Greifwerkzeugs zum Greifen des Stechmittels. Darüber hinaus ist es vorteilhaft, wenn die Vorrichtung eine Aufbringeinrichtung für das Klebemittel, eine zweite und/oder eine erste Temperiereinrichtung umfasst. Weiterhin ist es von Vorteil, wenn die Vorrichtung eine Temperaturmesseinrichtung, eine Steuereinrichtung und eine Aushärteeinrichtung umfasst.

Weitere Vorteile, Ziele und Eigenschaften der vorliegenden Erfindung werden anhand nachfolgender Beschreibung der anliegenden Figuren erläutert. Gleichartige Komponenten können in den verschiedenen Ausführungsformen gleiche Bezugszeichen aufweisen.

In den Figuren zeigen:
- Fig.1: einen bevorzugten Abschnitt eines Verfahrens zum Verbinden eines ersten Bauteils, insbesondere eines Stechmittels, mit einem zweiten Bauteil, insbesondere eines Spritzenkörpers, nach einer Ausführungsform;
- Fig.2: einen bevorzugten Abschnitt eines Verfahrens zum Verbinden eines ersten Bauteils, insbesondere eines Stechmittels, mit einem zweiten Bauteil, insbesondere eines Spritzenkörpers, nach einer weiteren Ausführungsform;
- Fig.3: einen bevorzugten Abschnitt eines Verfahrens zum Verbinden eines ersten Bauteils, insbesondere eines Stechmittels, mit einem zweiten Bauteil, insbesondere eines Spritzenkörpers, nach einer weiteren Ausführungsform;
- Fig.4: einen bevorzugten Abschnitt eines Verfahrens zum Verbinden eines ersten Bauteils, insbesondere eines Stechmittels, mit einem zweiten Bauteil, insbesondere eines Spritzenkörpers, nach einer weiteren Ausführungsform;
- Fig.5: einen bevorzugten Abschnitt eines Verfahrens zum Verbinden eines ersten Bauteils, insbesondere eines Stechmittels, mit einem zweiten Bauteil, insbesondere eines Spritzenkörpers, nach einer weiteren Ausführungsform;
- Fig.6: einen bevorzugten Abschnitt eines Verfahrens zum Verbinden eines ersten Bauteils, insbesondere eines Stechmittels, mit einem zweiten Bauteil, insbesondere eines Spritzenkörpers, nach einer weiteren Ausführungsform;
- Fig.7: ein Produkt hergestellt nach dem Verfahren zum Verbinden eines ersten Bauteils, insbesondere eines Stechmittels, mit einem zweiten Bauteil, insbesondere eines Spritzenkörpers, nach einer weiteren Ausführungsform;
- Fig.8: einen bevorzugten Abschnitt eines Verfahrens zum Verbinden eines ersten Bauteils, insbesondere eines Stechmittels, mit einem zweiten Bauteil, insbesondere eines Spritzenkörpers nach einer weiteren Ausführungsform;
- Fig.9: einen bevorzugten Abschnitt eines Verfahrens zum Verbinden eines ersten Bauteils, insbesondere eines Stechmittels, mit einem zweiten Bauteil, insbesondere eines Spritzenkörpers nach einer weiteren Ausführungsform.

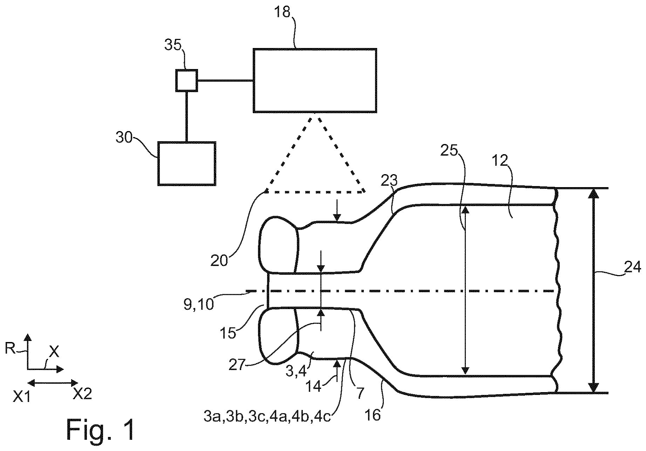

In den Figuren 1 bis 9 sind Ausführungsformen eines Verfahrens zum Verbinden eines ersten Bauteils (1), mit einem zweiten Bauteil (3) dargestellt. In den Figuren und im Folgenden ist das erste Bauteil (1) ein Stechmittel (2) und das zweite Bauteil (3) ein Spritzenkörper (4). Dies ist jedoch ohne Beschränkung der Allgemeinheit der Begriffe "erstes Bauteil (1)" und "zweites Bauteil (3)" zu verstehen. Das Verfahren ist analog auf anderweitige Bauteile anwendbar. Im Folgenden sind die Begriffe erstes Bauteil (1) und Stechmittel (2) demnach synonym zu verstehen. Ebenso sind die Begriffe zweites Bauteil (1) und Spritzenkörper (2) synonym zu verstehen.

Das Verfahren zum Verbinden eines ersten Bauteils (1), insbesondere eines Stechmittels (2), mit einem zweiten Bauteil (3), insbesondere eines Spritzenkörpers (4), zur Herstellung eines Produkts (5) für medizinische oder kosmetische Zwecke, mittels Kleben, umfasst die folgenden Schritte:
a) Bereitstellen des ersten (1, 2) und des zweiten Bauteils (3, 4)
b) Temperieren zumindest einer ersten Kontaktoberfläche (6) des ersten Bauteils (1, 2) und/oder zumindest einer zweiten Kontaktoberfläche (7) des zweiten Bauteils (3, 4) auf eine Temperatur T1;
c) Aufbringen eines Klebemittels (8) auf zumindest einen Abschnitt der ersten Kontaktoberfläche (6) und/oder auf zumindest einen Abschnitt der zweiten Kontaktoberfläche (7), wobei das Klebemittel (8) eine Temperatur T2 aufweist, wobei die Temperaturen T1 und T2 sich lediglich durch eine Toleranzabweichung unterscheiden, wobei die Toleranzabweichung maximal 10°C ist.

Die Temperatur T2 ist in einem Temperaturbereich zwischen 39°C und 46°C. Bevorzugt weicht die Temperatur T1 von der Temperatur T2 um maximal 5°C ab. Weiter bevorzugt weicht die Temperatur T1 von der Temperatur T2 um maximal 4°C ab. Weiter bevorzugt weicht die Temperatur T1 von der Temperatur T2 um maximal 3°C ab. Weiter bevorzugt weicht die Temperatur T1 von der Temperatur T2 um maximal 1,5°C ab.

Die Erfindung sieht demnach vor, einen oder beide Klebepartner (1, 2, 3, 4) auf die gleiche oder ähnliche Temperatur wie den verwendeten Klebstoff (8) zu temperieren. Dadurch kommt es nicht zu einem Temperaturausgleich beim Zusammentreffen der Klebepartner (1, 2, 3, 4) und des Klebstoffes (8). Der Klebstoff (8) behält seine gewünschte Temperatur und damit auch die gewünschte Viskosität.

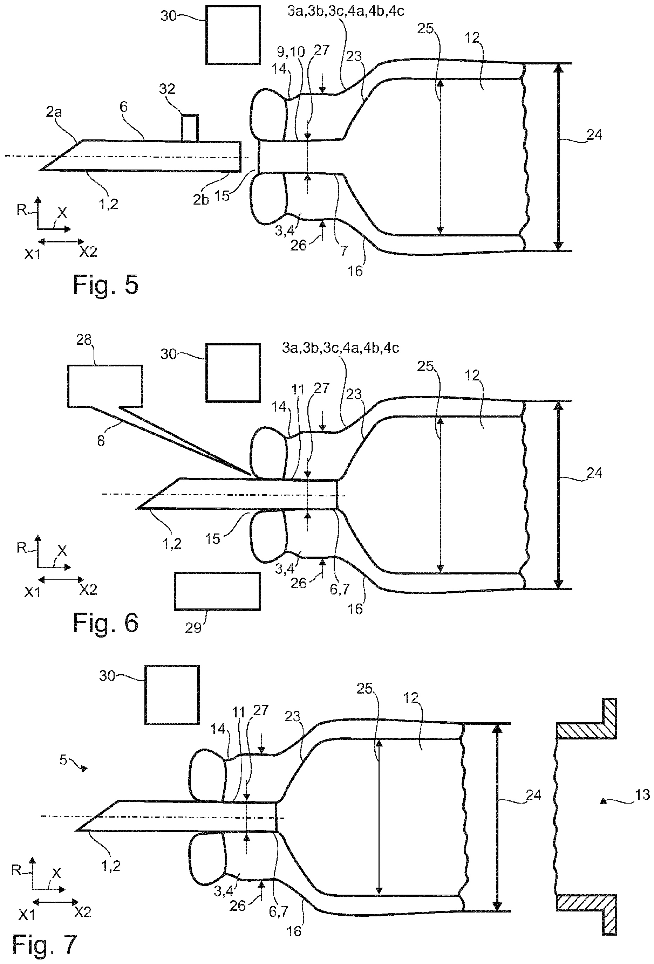

Ein entsprechendes Produkt (1), hergestellt durch das Verfahren, beziehungsweise ein Spritzenkörper (4) mit einem Stechmittel (2), ist in der Figur 7 dargestellt.

Der Spritzenkörper (4) erstreckt sich entlang einer axialen Achse und weist einen Vorratsbehälter (12) auf, in welchem ein zur Verabreichung vorgesehenes Medium aufbewahrbar ist. Die axiale Achse weist weiterhin eine distale Richtung X1 und eine proximale Richtung X2 auf. Der Vorratsbehälter (12) umfasst weiterhin eine proximale Öffnung (13) des Spritzenkörpers (4). Durch diese proximale Öffnung (18) kann das Medium in den Vorratsbehälter (12) gefüllt werden und im Anschluss der Kolbenstopfen eingesetzt werden. Der Spritzenkörper (4) weist weiterhin einen distalen Endbereich (14) auf, in welchem ein Stechmittelkanal (10) vorgesehen ist, wobei der Stechmittelkanal (10) einem Aufnahmevolumen (9) des zweiten Bauteils (3) entspricht. Der distale Endbereich (14) wird auch als Spritzenkonus bezeichnet. Der Stechmittelkanal (10) ist mit dem Vorratsbehälter (12) verbunden, so dass ein Medium aus dem Vorratsbehälter (12) in den Stechmittelkanal (10) treten kann.

Der distale Endbereich (14) weist weiterhin eine distale Öffnung (15) des Spritzenkörpers (4) auf. Diese distale Öffnung (15) ist gleichzeitig die distale Öffnung (15) des Stechmittelkanals (10). Schließlich weist der Spritzenkörper (4) einen Übergangsbereich (16) auf, der zwischen dem distalen Endbereich (14) und dem Vorratsbehälter (12) angeordnet ist.

Der Vorratsbehälter (12) weist einen ersten Außendurchmesser (25) und einen ersten Innendurchmesser (26) auf. Der distale Endbereich (14) weist einen zweiten Außendurchmesser (26) und einen zweiten Innendurchmesser (27) auf. Der zweite Außendurchmesser (26) ist kleiner als der erste Außendurchmesser (24) und der zweite Innendurchmesser (27) ist kleiner als der erste Innendurchmesser (25). Im Übergangsbereich (16) geht der erste Innendurchmesser (25) in den zweiten Innendurchmesser (27) über. Ebenso geht in dem Übergangsbereich (16) der erste Außendurchmesser (24) in den zweiten Außendurchmesser (26) über.

Zum Temperieren der ersten Kontaktoberfläche (6) des ersten Bauteils (1) beziehungsweise des Stechmittels (2) in Schritt b) wird ein erstes Temperiermedium (19) verwendet, welches durch eine erste Temperiereinrichtung (17) dem Stechmittel (1, 2) zugeführt wird. Zum Temperieren der zweiten Kontaktoberfläche (7) des zweiten Bauteils (3) beziehungsweise des Spritzenkörpers (4) wird ein zweites Temperiermedium (20) verwendet, welches durch eine zweite Temperiereinrichtung (18) dem Spritzenkörper (3, 4) zugeführt wird. Das erste (19) und/oder das zweite Temperiermedium (20) können ein Fluid, elektromagnetische Strahlung oder ein Plasma sein. Demnach könnte die erste (17) und/oder die zweite Temperiereinrichtung (18) ein Heißluftgebläse ein Heizstrahler, eine Warmwasserpumpe oder Ähnliches sein.

Die Temperierung in Schritt b) kann direkt oder indirekt erfolgen. Bei der direkten Temperierung trifft ein Temperiermedium (19, 20) unmittelbar auf die zumindest eine erste Kontaktoberfläche (6) und/oder die zumindest eine zweite Kontaktoberfläche (7). Bei der indirekten Temperierung trifft ein Temperiermedium auf einen Temperierbereich (1a, 3a) des ersten Bauteils (1) und/oder zweiten Bauteils (3), wobei der Temperierbereich (1a, 3a) des ersten Bauteils (1) und/oder zweiten Bauteils (3a) die Kontaktoberfläche (6, 7) des jeweiligen Bauteils (1, 3) umfasst, umgibt oder in unmittelbarer Nähe zu dieser Kontaktoberfläche (6, 7) angeordnet ist. Bei der indirekten Temperierung trifft ein Temperiermedium (19, 20) auf eine Temperieroberfläche (3b, 4b) des Temperierbereichs (3a, 4a), welche nicht der Kontaktoberfläche entspricht. Vorteilhafterweise erfolgt ein Wärmefluss von der Temperiereoberfläche (3b, 4b) durch den Temperierbereich (3a, 4a) zu der entsprechenden Kontaktoberfläche (6, 7) oder ein Wäremefluss von der entsprechenden Kontaktoberfläche (6, 7) zu der Temperieroberfläche (3a, 4a). Durch die Richtung des Wärmeflusses wird vorgegeben, ob die entsprechende Kontaktoberfläche (6, 7) erwärmt oder gekühlt wird.

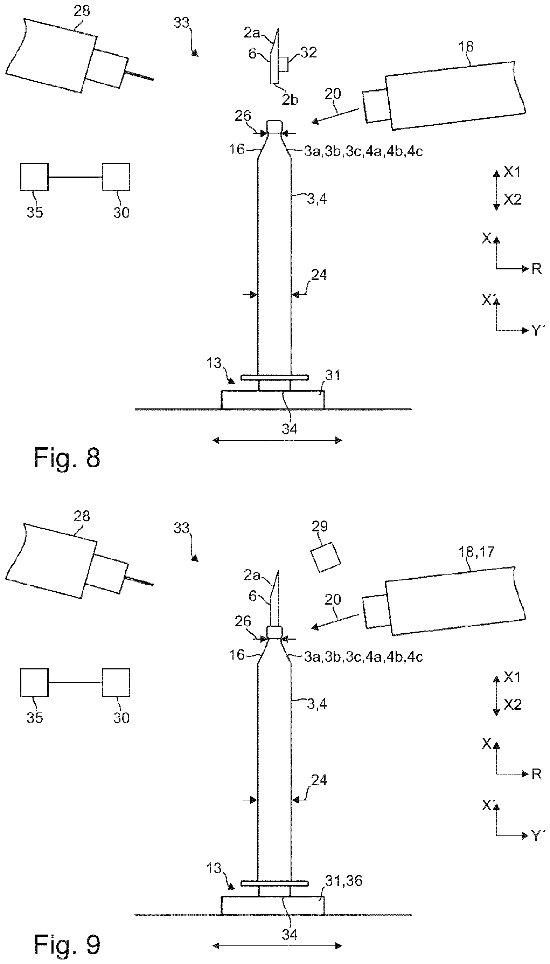

In Ausführungsformen nach den Figuren 1 und 8 wird die zweite Kontaktoberfläche (7) indirekt temperiert, wobei zumindest ein Abschnitt einer äußeren Oberfläche (3a, 4a) des distalen Endbereichs (14) mittels einer zweiten Temperiereinrichtung (18) temperiert wird, wobei mittels einer Wärmediffusion von der äußeren Oberfläche (3a, 4a) des distalen Endbereichs (14) zu der zweiten Kontaktoberfläche (7) oder von der zweiten Kontaktoberfläche (7) zu der äußeren Oberfläche (3a, 4a) des distalen Endbereichs (14) die zweite Kontaktoberfläche (7) temperiert wird.

Der Abschnitt der äußeren Oberfläche (3c, 4c) des distalen Endbereichs (14) entspricht somit der Temperieroberfläche (3b, 4b). Da der Stechmittelkanal (10) kreiszylindrisch ausgestaltet ist, ist die zweite Konaktoberfläche (7) die innere Oberfläche dieses Hohlzylinders. Vorteilhafterweise ist der distale Endbereich (14) der Temperierbereich. Dabei ist es vorteilhaft, wenn der gesamte distale Endbereich (14) derart temperiert wird, dass diese die Temperatur T1 aufweist.

Eine weitere Ausgestaltung der indirekten Temperierung ist in Figur 9 dargestellt. Dabei ist das erste Bauteil (1) beziehungsweise das Stechmittel (2) bereits vor der Temperierung in Schritt b) in das Aufnahmevolumen (9) beziehungsweise den Stechmittelkanal (10) eingeführt. Durch die Temperiereinrichtung (17, 18) wird analog zu der Ausführungsform zu Figur 8 zumindest ein Abschnitt einer äußeren Oberfläche (3a, 4a) des distalen Endbereichs (14) temperiert. Da das Stechmittel (2) sich bereits in dem Stechmittelkanal (10) befindet wird auch die erste Kontaktoberfläche (6) des Stechmittels (2) durch die Warmediffusion von oder zu der äußeren Oberfläche (3a, 4a) temperiert.

Alternativ oder kummulativ kann das Temperiermedium (19, 20) auf einen Abschnitt des Stechmittels (2) treffen, welcher über die distale Öffnung (15) des Spritzenkörpers hinausragt. Dieser Abschnitt wäre dann der Temperierbereich (1a) des ersten Bauteils (1). Durch die Wärmedissusion von oder zu diesem Temperierbereich (1a) wird dann die erste (6) und/oder die zweite Kontaktoberfläche (7) temperiert.

Die Temperiereinrichtung (17, 18) weist eine Öffnung auf, welche in radialer Richtung von dem distalen Endbereich (14) und/oder dem Stechmittel (2) beabstandet ist. Das Temperiermedium (19, 20) tritt aus dieser Öffnung auf und trifft auf die jeweilige äußere Oberfläche des Temperierbereichs (1a, 3a, 4a).

Ferner wäre es möglich, dass die Temperiereinrichtung (17, 18) zumindest zwei Öffnungen aufweist, welche in unterschiedlichen radialen Richtungen von dem distalen Endbereich (14) und/oder dem Stechmittel (2) beabstandet sind. Beispielsweise wäre es möglich zwei solcher Öffnungen diametral gegenüber anzuordnen. Das Temperiermedium kann somit auf zwei sich diametral gegenüberliegenden Abschnitten des distalen Endbereichs (14) und/oder des Stechmittels (2) auftreffen.

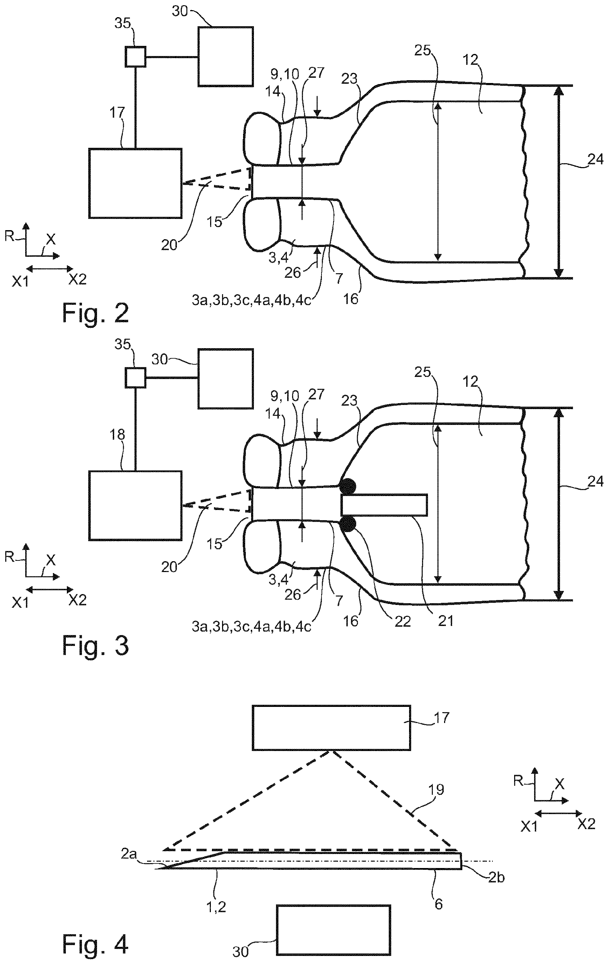

Nach den Ausführungsformen gemäß den Figuren 2, 3 und 4 werden die Kontaktoberflächen (6, 7) direkt temperiert.

In Figur 4 beispielsweise wird eine direkte Temperierung der ersten Kontaktfläche (6) des ersten Bauteils (1) beziehungsweise des Stechmittels (2) gezeigt. Das Temperiermedium (19) wird dabei von der ersten Temperiereinrichtung (17) direkt auf die erste Kontaktfläche aufgebracht.

In den Figuren 2 und 3 wird eine direkte Temperierung der zweiten Kontaktfläche (7) des zweiten Bauteils (3) beziehungsweise des Spritzenkörpers (4) insbesondere des distalen Endabschnitts (14) gezeigt. Gemäß dieser Ausführungsform wird ein zweites Temperiermedium (20), durch die zweite Temperiereinrichtung (18) in den Stechmittelkanal (10) des Spritzenkörpers (4) eingebracht. Eine solche Temperiereinrichtung (18) kann eine beliebige geeignete Einrichtung sein, welche ein geeignetes Temperiermedium (20) in den Stechmittelkanal (10) einbringen kann. Die zweite Temperiereinrichtung (18), beziehungsweise eine Austrittsöffnung der zweiten Temperiereinrichtung wird an einer distalen Öffnung (15) des Stechmittelkanals (10) platziert, so dass das Temperiermedium (20) in proximaler Richtung X2 in den Stechmittelkanal (9, 10) eindringt. Dort trifft es dann direkt auf die zweite Kontaktoberfläche (7), wodurch diese temperiert wird.

Aufgrund der hohen Reinheitsanforderungen bei Spritzenkörpern ist es oftmals erstrebenswert, dass das Temperiermedium (19, 20) nicht in den Vorratsbehälter (12) des Spritzenkörpers (4) gelangt. Demnach kann bei der direkten Temperierung des Spritzenkörpers (4) eine Abführeinrichtung (21) vorgesehen werden, welche das zweite Temperiermedium (20) aus dem Spritzenköper (4) entfernt. Eine solche Abführeinrichtung (21) ist schematisch in Figur 3 dargestellt und kann eine Absaugeeinrichtung sein. Die Abführeinrichtung (21) ist mit einer Dichtungseinrichtung (22) ausgestattet, welche an einer Innenwand (23) des Übergangsbereich (16) dichtend anliegt. Durch die Abführeinrichtung (21) und die Dichtungseinrichtung (22) wird verhindert, dass ein Temperiermedium (19, 20) in den Vorratsbehälter (12) gelangt. Die Abführeinrichtung (21) wird dabei durch die proximale Öffnung (13) des Spritzenkörper (3, 4) eingeführt. Es wäre auch denkbar, dass die Abführeinrichtung (21) in der ersten Transporteinrichtung (31), welche das zweite Bauteil (3), beziehungsweise den Spritzenkörper transportiert, angeordnet ist.

Sowohl bei der direkten als auch bei der indirekten Temperierung können also lediglich das Stechmittel oder lediglich der Spritzenkörper temperiert werden. Möglich wäre es auch, dass das Stechmittel (1, 2) und der Spritzenkörper (3, 4) durch eine oder mehrere Temperiereinrichtung(en) (17, 18) temperiert werden.

Nach Schritt b), also dem Temperieren zumindest einer ersten Kontaktoberfläche (6) des ersten Bauteils (1, 2) und/oder zumindest einer zweiten Kontaktoberfläche (7) des zweiten Bauteils (3, 4) auf eine Temperatur T1, wird vorteilhafterweise das Stechmittel (1, 2) in den Stechmittelkanal (9, 10) des Spritzenkörpers (3, 4) zumindest abschnittsweise eingeführt. Das Stechmittel (2) weist ein proximales Ende (2b) und ein distales Ende (2a) auf, welches mit einem Schliff versehen ist. Das proximale Ende (2b) des Stechmittels (1, 2) wird in proximaler Richtung X2 in den Stechmittelkanal (9, 10) eingeführt. Ein gewisser Abschnitt des Stechmittels mit dem distalen Ende (2a) ragt demnach über die distale Öffnung (15) des Spritzenkörpers (4) hinaus. Dies ist in den Figuren 5 bis 6 dargestellt. Das proximale Ende (2b) des Stechmittels (1, 2) ragt bei dieser Ausführungsform in proximaler Richtung (X2) nicht über den Stechmittelkanal (9, 10) hinaus. Es wäre jedoch auch möglich, dass das Stechmittel derart in den Spritzenkörper eingeführt wird, dass proximale Ende (2b) des Stechmittels (1, 2) in den Übergangsbereich (16) hineinragt.

Der Klebeholraum (11) wird demnach durch das Restvolumen des Stechmittelkanals (9, 10) gebildet, welches nicht durch das Stechmittel (1, 2) ausgefüllt wird.

In Schritt c) wird dann das Klebemittel (8) auf die gesamte erste Kontaktoberfläche (6) und die gesamte zweite Kontaktoberfläche (7) aufgebracht. Dies erfolgt durch das Ausfüllen des Klebeholraums (11) mit Klebemittel (8). Dies wird in Figur 6 gezeigt. Das Klebemittel (8) wird durch eine Aufbringeinichtung (28) durch die distale Öffnung des Spritzenkörpers (4) in den Stechmittelkanal (9, 10) beziehungsweise den Klebehohlraum (11) eingebracht, so dass der Klebehohlraum (11) vollständig mit Klebemittel (8) befüllt ist und die gesamten Kontaktoberflächen (6, 7) mit Klebemittel bedeckt sind. Nach dem Aushärten des Klebemittels besteht somit eine Klebeverbindung zwischen der gesamten ersten Kontaktoberfläche (6) und der gesamten zweiten Kontaktoberfläche (7).

Alternativ wird nach Schritt c), also dem Aufbringen eines Klebemittels (8) auf zumindest einen Abschnitt der ersten Kontaktoberfläche (6) und/oder auf zumindest einen Abschnitt der zweiten Kontaktoberfläche (7), das Stechmittel (1, 2) in den Stechmittelkanal (9, 10) zumindest abschnittsweise eingeführt. Das Stechmittel (1, 2) wird dabei in proximaler Richtung X2 in den Stechmittelkanal (9, 10) eingeführt. Dies ist in Figur 5 dargestellt. Zuvor wird in Schritt c) das Klebemittel (8) auf zumindest einen Abschnitt der ersten Kontaktoberfläche (6) und/oder auf zumindest einen Abschnitt der zweiten Kontaktoberfläche (7) aufgebracht. Die aufgebrachte Menge an Klebemittel (8) ist dabei derart bemessen, dass der Klebehohlraum (11) ausgefüllt ist, wenn das Stechmittel in den Stechmittelkanal (9, 10) eingeführt worden ist.

Es wäre denkbar, eine Temperaturmesseinrichtung (30) vorzusehen, welche die Temperatur T1 und/oder die Temperatur T2 überwacht. Eine solche Temperaturmessung könnte kontaktlos oder mit einem Temperaturfühler durchgeführt werden. Denkbar wäre es, lediglich die Temperatur einer äußeren Oberfläche des distalen Endbereichs (14) durchzuführen. Durch die Abmessungen des distalen Endbereichs (14) und durch die Vorgabe des Materials kann dann auf die Temperatur T1 der zweiten Kontaktoberfläche geschlossen werden. Die Temperaturmesseinrichtung (30) kann mit einer Steuereinrichtung (35) verbunden sein, welche den gemessenen Wert mit dem vorgegebenen Sollwert vergleicht. Die Steuereinrichtung (35) kann dann die Temperiereinrichtungen (17, 18) entsprechend ansteuern.

Optional kann die erste Kontaktoberfläche (6) vor Schritt c) vorbehandelt und/oder aktiviert werden, wobei diese Vorbehandlung der ersten Kontaktoberfläche (6) ausgewählt ist aus der Gruppe Reinigung, mechanische Vorbehandlung, chemische Vorbehandlung, thermische Vorbehandlung, elektrochemische Vorbehandlung, physikalische Vorbehandlung. Ebenso kann, die zweite Kontaktoberfläche (7) vor Schritt c) vorbehandelt und/oder aktiviert werden, wobei die Vorbehandlung der zweiten Kontaktoberfläche (7) ausgewählt ist aus der Gruppe Reinigung, mechanische Vorbehandlung, chemische Vorbehandlung, thermische Vorbehandlung, elektrochemische Vorbehandlung, physikalische Vorbehandlung.

Das Klebemittel (8) kann durch eine geeignete Aushärteeinrichtung (29) ausgehärtet werden. Die Aushärteeinrichtung ist in radialer Richtung (R) neben dem distalen Endbereich (14) angeordnet.

In den Figuren, insbesondere in den Figuren 8 und ist auch eine Vorrichtung (33) zum Verbinden zweier Bauteile insbesondere von einer Vorrichtung (33) zum Bestücken eines Spritzenkörpers (4) mit einem Stechmittel (6) dargestellt. Die Vorrichtung (33) umfasst die vertikale Achse X', welche bevorzugt parallel zu der axialen Achse X des Spritzenkörpers (4) ist. Ferner umfasst die Vorrichtung eine horizontale Achse Y`.

Die Vorrichtung (33) umfasst eine erste Transporteinrichtung (31), welche den Spritzenkörper (4) zu einer Bearbeitungsposition zuführt. Die Transporteinrichtung umfasst eine Aufnahmeeinrichtung (34), in welcher das proximale Ende des Spritzenkörpers (4) aufgenommen wird. Die Vorrichtung (33) umfasst weiterhin eine Aufbringeinrichtung (28) für das Klebemittel (8). Diese Aufbringeinrichtung ist mit einem Klebemittelvorrat verbunden. Die Aufbringeinrichtung umfasst vorzugsweise eine Klebemitteltemperiereinrichtung, mittels welcher die Temperatur T2 des Klebemittels (8) einstellbar ist. Diese Klebemitteltemperiereinrichtung kann in dem Klebemittelvorrat integriert sein. Alternativ kann die jeweils entnommene Klebemittelmenge temperiert werden. Es ist auch denkbar, dass eine Temperaturmesseinrichtung vorgesehen ist, welche die Klebemitteltemperatur misst. Auch diese Temperaturmesseinrichtung kann mit der Steuereinrichtung (35) verbunden sein, welche die Klebemitteltemperatur mit dem vorgegebenen Wert vergleicht und die Klebemitteltemperiereinrichtung entsprechend ansteuert.

Die Vorrichtung (33) umfasst eine erste und/oder eine zweite Temperiereinrichtung, die Temperaturmesseinrichtung (30), die Steuereinrichtung und eine Aushärteeinrichtung (29). Ferner umfasst die Vorrichtung (33) eine zweite Transporteinrichtung (32) in Form eines Greifwerkzeugs zum Greifen des Stechmittels (6). Vorteilhafterweise ist das Stechmittel (6) mittels des Greifwerkzeugs (32) in den Stechmittelkanal (10) des Spritzenkörpers (4) einführbar.

In der Bearbeitungspostion ist der Spritzenkörper (4) und/oder das Stechmittel (2) derart platziert, dass die Temperiereinrichtung(en) (17, 18) und die Aufbringeinrichtung (28) für das Klebemittel (8), gegebenenfalls die Aushärteeienrichtung (29) entsprechend ihre Funktion ausführen können. Beispielsweise wäre denkbar, dass die Temperiereinrichtung(en) (17, 18) die Aufbringeinrichtung (28) für das Klebemittel (8), und gegebenenfalls die Aushärteeinrichtung (29) radial um eine Bearbeitungsposition angeordnet sind. Alternativ wäre denkbar, dass der Spritzenkörper (4) und/oder das Stechmittel (2) einer bestimmten Einrichtung (17, 18, 28, 29) nacheinander zugeführt werden. Somit würde für jede der genannten Einrichtungen eine Bearbeitungsposition existieren.

Sämtliche in den Anmeldungsunterlagen offenbarten Merkmale werden als erfindungswesentlich beansprucht, sofern sie einzeln oder in Kombination gegenüber dem Stand der Technik neu sind.

### Bezugszeichenliste

- 1: erstes Bauteil
- 1a: Temperierbereich des ersten Bauteils
- 2: Stechmittel
- 2a: distales Ende des Stechmittels
- 2b: proximales Ende des Stechmittels
- 3: zweites Bauteil
- 3a: Temperierbereich des zweiten Bauteils
- 3b: Temperieroberfläche
- 3c: äußere Oberfläche des zweiten Bauteils
- 4: Spritzenkörper
- 4a: Temperierbereich des Spritzenkörpers
- 4b: Temperieroberfläche
- 4c: äußere Oberfläche des Spritzenkörpers
- 5: Produkt
- 6: erste Kontaktoberfläche des ersten Bauteils
- 7: zweite Kontaktoberfläche des zweiten Bauteils
- 8: Klebemittel
- 9: Aufnahmevolumen
- 10: Stechmittelkanal
- 11: Klebehohlraum
- 12: Vorratsbehälter
- 13: proximale Öffnung des Spritzenkörpers
- 14: distalen Endbereich des Spritzenkörpers
- 15: distale Öffnung des Spritzenkörpers
- 16: Übergangsbereich des Spritzenkörpers
- 17: erste Temperiereinrichtung
- 18: zweite Temperiereinrichtung
- 19: erstes Temperiermedium
- 20: zweites Temperiermedium
- 21: Abführeinrichtung
- 22: Dichtungseinrichtung
- 23: Innenwand des Übergangsbereichs
- 24: erster Außendurchmesser des Vorratsbehälters
- 25: erster Innendurchmesser des Vorratsbehälters
- 26: zweiter Außendurchmesser des distalen Endbereichs
- 27: zweiter Innendurchmesser des distalen Endbereichs
- 28: Aufbringeinrichtung
- 29: Aushärteeinrichtung
- 30: Temperaturmesseinrichtung
- 31: erste Transporteinrichtung
- 32: zweite Transporteinrichtung
- 33: Vorrichtung zum Verbinden zweier Bauteile
- 34: Aufnahmeeinrichtung
- 35: Steuereinrichtung
- X: axiale Achse
- X1: distale Richtung
- X2: proximale Richtung
- R: radiale Richtung
- X': Vertikale Achse der Vorrichtung zum Verbinden zweier Bauteile
- Y': horizontale Achse der Vorrichtung zum Verbinden zweier Bauteile

## Patentansprüche

1. Verfahren zum Verbinden eines ersten Bauteils (1), nämlich eines Stechmittels (2), mit einem zweiten Bauteil (3), nämlich eines Spritzenkörpers (4), zur Herstellung eines Produkts (5) für medizinische oder kosmetische Zwecke, mittels Kleben, umfassend die folgenden Schritte:
a) Bereitstellen des ersten (1, 2) und des zweiten Bauteils (3, 4)
b) Temperieren zumindest einer ersten Kontaktoberfläche (6) des ersten Bauteils (1, 2) und zumindest einer zweiten Kontaktoberfläche (7) des zweiten Bauteils (3, 4) auf eine Temperatur T1;
c) Aufbringen eines Klebemittels (8) auf zumindest einen Abschnitt der ersten Kontaktoberfläche (6) und/oder auf zumindest einen Abschnitt der zweiten Kontaktoberfläche (7), wobei das Klebemittel (8) eine Temperatur T2 aufweist, wobei die Temperaturen T1 und T2 sich lediglich durch eine Toleranzabweichung unterscheiden, wobei die Toleranzabweichung maximal 10°C ist, wobei die Temperatur T2 größer als die Raumtemperatur ist,
wobei das zweite Bauteil (3) ein Aufnahmevolumen (9, 10) aufweist, welches zumindest abschnittsweise von der zweiten Kontaktoberfläche (7) begrenzt ist, wobei das erste Bauteil (1, 2) abschnittsweise in dem Aufnahmevolumen (9, 10) anordenbar ist, wobei bei einer Anordnung des ersten Bauteils (1, 2) in dem Aufnahmevolumen (9, 10) sich die erste (6) und die zweite Kontaktoberfläche (7) gegenüberliegen, wobei bei einer Anordnung des ersten Bauteils (1, 2) in dem Aufnahmevolumen (9, 10) zumindest abschnittsweise zwischen den Kontaktoberflächen (6, 7) ein Klebehohlraum (11) ausgebildet ist, **dadurch gekennzeichnet, dass** das Klebemittel ein Urethanmethacrylat ist, das bei 25°C eine Viskosität von 1000 bis 2000 mPa s aufweist und bei der Temperatur T2 eine benötigte Viskosität aufweist, wobei eine ausreichende Erniedrigung der Viskosität um den Klebehohlraum zu füllen, in einem Temperaturbereich zwischen 39°C und 46°C liegt.

2. Verfahren nach Anspruch 1,
**dadurch gekennzeichnet, dass**
die Temperierung in Schritt b) direkt oder indirekt erfolgen kann, wobei bei der direkten Temperierung ein Temperiermedium (19, 20) unmittelbar auf die zumindest eine erste Kontaktoberfläche (6) und/oder die zumindest eine zweite Kontaktoberfläche (7) trifft, wobei bei der indirekten Temperierung ein Temperiermedium auf einen Temperierbereich (1a, 3a, 4a) des ersten Bauteils (1, 2) und/oder zweiten Bauteils (3, 4) trifft, wobei der Temperierbereich (1a, 3a, 4a) des ersten Bauteils (1, 2) und/oder zweiten Bauteils (3, 4) die Kontaktoberfläche (6, 7) des jeweiligen Bauteils (1, 2, 3, 4) umfasst, umgibt oder in unmittelbarer Nähe zu dieser Kontaktoberfläche (6, 7) angeordnet ist.

3. Verfahren nach einem der Ansprüche 1 oder 2,
**dadurch gekennzeichnet, dass**
nach Schritt b) das erste Bauteil (1) in das Aufnahmevolumen (9, 10) des zweiten Bauteils (3, 4) zumindest abschnittsweise eingeführt wird, wobei in Schritt c) das Klebemittel (8) auf die gesamte erste Kontaktoberfläche (6) und die gesamte zweite Kontaktoberfläche (7) aufgebracht wird, in dem der Klebeholraum (11) mit Klebemittel (8) ausgefüllt wird.

4. Verfahren nach einem der Ansprüche 1 oder 2,
**dadurch gekennzeichnet, dass**
nach Schritt c) das erste Bauteil (1, 2) in das Aufnahmevolumen (9, 10) des zweiten Bauteils (3, 4) zumindest abschnittsweise eingeführt wird, wobei in Schritt c) das Klebemittel (8) auf zumindest einen Abschnitt der ersten Kontaktoberfläche (6) oder auf zumindest einen Abschnitt der zweiten Kontaktoberfläche (7) aufgebracht wird, wobei die aufgebrachte Menge an Klebemittel (8) derart bemessen ist, dass der Klebehohlraum (11) ausgefüllt ist.

5. Verfahren nach einem der vorhergehenden Ansprüche,
**dadurch gekennzeichnet, dass**
wobei die Temperatur T1 von der Temperatur T2 um maximal 5°C abweicht, wobei die Temperatur T1 von der Temperatur T2 um maximal 3°C abweicht.

6. Verfahren nach einem der vorhergehenden Ansprüche,
**dadurch gekennzeichnet, dass**
die erste Kontaktoberfläche (6) vor Schritt c) vorbehandelt und/oder aktiviert wird, wobei diese Vorbehandlung der ersten Kontaktoberfläche (6) ausgewählt ist aus der Gruppe Reinigung, mechanische Vorbehandlung, chemische Vorbehandlung, thermische Vorbehandlung, elektrochemische Vorbehandlung, physikalische Vorbehandlung, wobei die zweite Kontaktoberfläche (7) vor Schritt c) vorbehandelt und/oder aktiviert wird, wobei die Vorbehandlung der zweiten Kontaktoberfläche (7) ausgewählt ist aus der Gruppe Reinigung, mechanische Vorbehandlung, chemische Vorbehandlung, thermische Vorbehandlung, elektrochemische Vorbehandlung, physikalische Vorbehandlung.

7. Verfahren nach einem der vorhergehenden Ansprüche,
**dadurch gekennzeichnet, dass**
zum Temperieren des ersten Bauteils (1, 2) ein erstes Temperiermedium (19) verwendet wird, wobei zum Temperieren des zweiten Bauteils (3, 4) ein zweites Temperiermedium (20) verwendet wird, wobei das erste (19) und/oder das zweite Temperiermedium (20) ein Fluid oder elektromagnetische Strahlung sein können, wobei das erste Temperiermedium (19) durch eine erste Temperiereinrichtung (17) dem ersten Bauteil (1, 2) zugeführt wird, wobei das zweite Temperiermedium (20) durch eine zweite Temperiereinrichtung (18) dem zweiten Bauteil (3, 4) zugeführt wird.

8. Verfahren nach Anspruch nach einem der vorhergehenden Ansprüche,
**dadurch gekennzeichnet, dass**
die zweite Temperiereinrichtung (18) und eine Aufbringeinrichtung (28) für das Klebemittel (8) in einer Vorrichtung zum Verbinden zweier Bauteile stationär angeordnet sind, wobei das zweite Bauteil (3, 4) durch eine erste Transporteinrichtung (31) zu einer Bearbeitungsposition von der zweiten Temperiereinrichtung (18) und/oder von der Aufbringeinrichtung (28) für das Klebemittel (8) geführt wird, wobei eine zweite Transporteinrichtung (32) das erste Bauteil (1, 2) in das zweite Bauteil (3, 4) einführt oder an das zweite Bauteil (3, 4) anfügt.

9. Verfahren nach einem der vorhergehenden Ansprüche,
**dadurch gekennzeichnet, dass**
das erste Bauteil (1) ein Stechmittel (2) ist und das zweite Bauteil (3) ein Spritzenkörper (4) ist, wobei der Spritzenkörper (4) einen Vorratsbehälter (12) aufweist, in welchem ein zur Verabreichung vorgesehenes Medium aufbewahrbar ist, wobei der Vorratsbehälter (12) eine proximale Öffnung (13) des Spritzenkörpers (4) aufweist, wobei der Spritzenkörper (4) einen distalen Endbereich (14) aufweist, in welchem ein Stechmittelkanal (10) vorgesehen ist, wobei der Stechmittelkanal (10) dem Aufnahmevolumen (9) des zweiten Bauteils (3) entspricht, wobei der distale Endbereich (14) eine distale Öffnung (15) des Spritzenkörpers (4) aufweist, wobei der Spritzenkörper (4) einen Übergangsbereich (16) aufweist, der zwischen dem distalen Endbereich (14) und dem Vorratsbehälter (12) angeordnet ist.

10. Verfahren nach Anspruch 9,
**dadurch gekennzeichnet, dass**
die zweite Kontaktoberfläche (7) indirekt temperiert wird, wobei zumindest ein Abschnitt einer äußeren Oberfläche (3b, 3c, 4b, 4c) des distalen Endbereichs (14) mittels einer zweiten Temperiereinrichtung (18) temperiert wird, wobei mittels einer Wärmediffusion von der äußeren Oberfläche (3b, 3c, 4b, 4c) des distalen Endbereichs (14) zu der zweiten Kontaktoberfläche (7) oder von der zweiten Kontaktoberfläche (7) zu der äußeren Oberfläche (3b, 3c, 4b, 4c) des distalen Endbereichs (14) die zweite Kontaktoberfläche (7) temperiert wird.

11. Verfahren nach Anspruch 9
**dadurch gekennzeichnet, dass**
die zweite Kontaktoberfläche (7) direkt temperiert wird, wobei ein zweites Temperiermedium (20), durch eine zweite Temperiereinrichtung (18) in den Stechmittelkanal (10) des distalen Endbereichs (4) eingebracht wird, wobei die Temperiereinrichtung (18) an einer distalen Öffnung (15) des Stechmittelkanals (10) platziert wird, wobei das zweite Temperiermedium (20) durch eine Abführeinrichtung (21) aus dem Spritzenköper (4) entfernt wird, wobei die Abführeinrichtung (21) in dem Übergangsbereich (16) angeordnet ist, wobei die Abführeinrichtung (21) eine Dichtungseinrichtung (22) umfasst, welche dichtend an einer Innenwand (23) des Übergangsbereichs (16) angeordnet ist, so dass kein Temperiermedium (20) in den Vorratsbehälter (12) gelangt.

## Claims

1. Method for connecting a first component (1), in form of a lancing means (2), to a second component (3), in form of a syringe body (4), in order to manufacture a product (5) for medical or cosmetic purposes, by means of adhesive bonding, comprising the following steps:
a) Providing the first (1, 2) and the second component (3, 4)
b) Tempering at least one first contact surface (6) of the first component (1, 2) and at least one second contact surface (7) of the second component (3, 4) to a temperature T1;
c) Applying an adhesive (8) to at least one portion of the first contact surface (6) and/or to at least one portion of the second contact surface (7), wherein the adhesive (8) is at a temperature T2, wherein temperatures T1 and T2 only differ by a tolerance deviation, wherein the tolerance deviation is at most 10°C, wherein temperature T2 is greater than the room temperature,
wherein the second component (3) has a receiving volume (9, 10) which is limited at least in sections by the second contact surface (7), wherein the first component (1, 2) can be arranged in sections in the receiving volume (9, 10),
wherein if the first component (1, 2) is arranged in the receiving volume (9, 10), the first (6) and the second contact surface (7) lie opposite one another, wherein, when the first component (1, 2) is arranged in the receiving volume (9, 10), an adhesive cavity (11) is formed at least in sections between the contact surfaces (6, 7), **characterized in that** the adhesive is a urethane methacrylate wherein at 25° C., such an adhesive has a viscosity of 1000 to 2000 mPa s and at temperature T2 has a required viscosity, wherein a sufficient reduction in the viscosity to fill the adhesive cavity is achieved in a temperature range between 39°C and 46°C.

2. Method according to claim 1,
**characterised in that**
the tempering in step b) can take place directly or indirectly, wherein, in the case of direct tempering, a tempering medium (19, 20) directly touches the at least one first contact surface (6) and/or at least one second contact surface (7), wherein, in the case of indirect tempering, a tempering medium touches a tempering region (1a, 3a, 4a) of the first component (1, 2) and/or second component (3, 4), wherein the tempering region (1a, 3a, 4a) of the first component (1, 2) and/or the second component (3, 4) comprises or surrounds the contact surface (6, 7) of the respective component (1, 2, 3, 4) or is arranged in the immediate vicinity of this contact surface (6, 7).

3. Method according to claims 1 or 2
**characterised in that**
after step b), the first component (1) is introduced at least in portions into the receiving volume (9, 10) of the second component (3, 4), wherein, in step c), the adhesive (8) is applied to the entire first contact surface (6) and the entire second contact surface (7) by filling the adhesive cavity (11) with adhesive (8).

4. Method according to claims 1 or 2,
**characterised in that**
after step c), the first component (1, 2) is introduced into the receiving volume (9, 10) of the second component (3, 4) at least in portions, wherein, in step c), the adhesive (8) is applied to at least one portion of the first contact surface (6) or to at least one portion of the second contact surface (7), wherein the amount of adhesive (8) applied is such that the adhesive cavity (11) is filled.

5. Method according to one of the preceding claims,
**characterized in that**,
wherein the temperature T1 deviates from the temperature T2 by a maximum of 5°C, wherein the temperature T1 deviates from the temperature T2 by a maximum of 3°C.

6. Method according to one of the preceding claims,
**characterised in that**
the first contact surface (6) is pretreated and/or activated before step c), wherein this pretreatment of the first contact surface (6) is selected from the group including cleaning, mechanical pretreatment, chemical pretreatment, thermal pretreatment, electrochemical pretreatment, physical pretreatment, wherein the second contact surface (7) is pretreated before step c), wherein the pretreatment of the second contact surface (7) is selected from the group including cleaning, mechanical pretreatment, chemical pretreatment, thermal pretreatment, electrochemical pretreatment, physical pretreatment.

7. Method according to one of the preceding claims,
**characterised in that**
a first tempering medium (19) is used for tempering the first component (1, 2), wherein a second tempering medium (20) is used for tempering the second component (3, 4), wherein the first (19) and/or the second tempering medium (20) can be a fluid or electromagnetic radiation, wherein the first tempering medium (19) is supplied to the first component (1, 2) by a first tempering device (17), wherein the second tempering medium (20) is supplied to the second component (3, 4) by a second tempering device (18).

8. Method according to one of the preceding claims,
**characterised in that**
the second tempering device (18) and an application device (28) for the adhesive (8) are stationarily arranged in an apparatus for connecting two components, wherein the second component (3, 4) is guided by a first transport device (31) to a processing position of the second tempering device (18) and/or of the application device (28) for the adhesive (8), wherein a second transport device (32) introduces the first component (1, 2) into the second component (3, 4) or attaches the same to the second component (3, 4).

9. Method according to one of the preceding claims,
**characterised in that**
the first component (1) is a lancing means (2) and the second component (3) is a syringe body (4), wherein the syringe body (4) has a reservoir (12) in which a medium provided for administration can be stored, wherein the reservoir (12) has a proximal opening (13) of the syringe body (4), wherein the syringe body (4) has a distal end region (14), in which a lancing means channel (10) is provided, wherein the lancing means channel (10) corresponds to the receiving volume (9) of the second component (3), wherein the distal end region (14) has a distal opening (15) of the syringe body (4), wherein the syringe body (4) has a transition region (16), which is arranged between the distal end region (14) and the reservoir (12).

10. Method according to claim 9,
**characterised in that**
the second contact surface (7) is tempered indirectly, wherein at least a portion of an outer surface (3b, 3c, 4b, 4c) of the distal end region (14) is tempered by means of a second tempering device (18), wherein the second contact surface (7) is tempered by means of thermal diffusion from the outer surface (3b, 3c, 4b, 4c) of the distal end region (14) to the second contact surface (7), or from the second contact surface (7) to the outer surface (3b, 3c, 4b, 4c) of the distal end region (14).

11. Method according to claim 9,
**characterised in that**
the second contact surface (7) is tempered directly, wherein a second tempering medium (20) is introduced by a second tempering device (18) into the lancing means channel (10) of the distal end region (4), wherein the tempering device (18) is placed at a distal opening (15) of the lancing means channel (10), wherein the second tempering medium (20) is removed from the syringe body (4) by a discharge device (21), wherein the discharge device (21) is arranged in the transition region (16), wherein the discharge device (21) comprises a sealing device (22) which is sealingly arranged on an inner wall (23) of the transition region (16), so that no tempering medium (20) enters the reservoir (12).

## Revendications

1. Procédé de liaison par collage d'un premier composant (1), à savoir d'un moyen piqueur (2), avec un second composant (3), à savoir un corps de seringue (4), pour la fabrication d'un produit (5) à usage médical ou cosmétique, ledit procédé comprenant les étapes suivantes :
a) préparer le premier (1, 2) et le second composant (3, 4) ;
b) réguler la température d'au moins une première surface de contact (6) du premier composant (1, 2) et d'au moins une seconde surface de contact (7) du second composant (3, 4) à une température T1 ;
c) appliquer un adhésif (8) sur au moins une section de la première surface de contact (6) et/ou sur au moins une section de la seconde surface de contact (7), l'adhésif (8) présentant une température T2, les températures T1 et T2 ne différant que par un écart de tolérance, l'écart de tolérance étant d'au plus 10°C, la température T2 étant supérieure à la température ambiante,
le second composant (3) présentant un volume de réception (9, 10), lequel est limité au moins par sections par la seconde surface de contact (7), le premier composant (1, 2) étant apte à être disposé par sections dans le volume de réception (9, 10), où, lorsque le premier composant (1, 2) est disposé dans le volume de réception (9, 10), la première (6) et la seconde (7) surface de contact se trouvent à l'opposé l'une de l'autre, où, lorsque le premier composant (1, 2) est disposé dans le volume de réception (9, 10), une cavité à adhésif (11) est formée au moins par sections entre les surfaces de contact (6, 7),
**caractérisé par le fait que**
l'adhésif est un méthacrylate d'uréthane qui présente une viscosité de 1000 à 2000 mPa.s à 25°C et présente une viscosité requise à la température T2, une diminution suffisante de la viscosité pour remplir la cavité à adhésif se situant dans une plage de température entre 39°C et 46°C.

2. Procédé selon la revendication 1,
**caractérisé par le fait que**
la régulation de température à l'étape b) peut avoir lieu directement ou indirectement, où, lors de la régulation directe de la température, un milieu de régulation de température (19, 20) frappe directement ladite au moins une première surface de contact (6) et/ou ladite au moins une seconde surface de contact (7), où, lors de la régulation indirecte de la température, un milieu de régulation de température frappe une région de régulation de température (1a, 3a, 4a) du premier composant (1, 2) et/ou du second composant (3, 4), la région de régulation de température (1a, 3a, 4a) du premier composant (1, 2) et/ou du second composant (3, 4) comprenant ou entourant la surface de contact (6, 7) du composant respectif (1, 2, 3, 4) ou étant disposée à proximité immédiate de cette surface de contact (6, 7).

3. Procédé selon l'une des revendications 1 ou 2,
**caractérisé par le fait qu'**
après l'étape b), le premier composant (1) est introduit au moins par sections dans le volume de réception (9, 10) du second composant (3, 4), où, à l'étape c) l'adhésif (8) est appliqué sur la totalité de la première surface de contact (6) et la totalité de la seconde surface de contact (7), par remplissage de la cavité à adhésif (11) par l'adhésif (8).

4. Procédé selon l'une des revendications 1 ou 2,
**caractérisé par le fait qu'**
après l'étape c), le premier composant (1, 2) est introduit au moins par sections dans le volume de réception (9, 10) du second composant (3, 4), où, à l'étape c) l'adhésif (8) est appliqué sur au moins une section de la première surface de contact (6) ou sur au moins une section de la seconde surface de contact (7), la quantité d'adhésif (8) appliquée étant mesurée de telle sorte que la cavité à adhésif (11) est remplie.

5. Procédé selon l'une des revendications précédentes,
**caractérisé par le fait que**
la température T1 s'écarte de la température T2 d'au plus 5°C, la température T1 s'écarte de la température T2 d'au plus 3°C.

6. Procédé selon l'une des revendications précédentes,
**caractérisé par le fait que**
la première surface de contact (6) est prétraitée et/ou activée avant l'étape c), ce prétraitement de la première surface de contact (6) étant choisi dans le groupe comprenant le nettoyage, le prétraitement mécanique, le prétraitement chimique, le prétraitement thermique, le prétraitement électrochimique, le prétraitement physique, la seconde surface de contact (7) étant prétraitée et/ou activée avant l'étape c), le prétraitement de la seconde surface de contact (7) étant choisi dans le groupe comprenant le nettoyage, le prétraitement mécanique, le prétraitement chimique, le prétraitement thermique, le prétraitement électrochimique, le prétraitement physique.

7. Procédé selon l'une des revendications précédentes,
**caractérisé par le fait qu'**
un premier milieu de régulation de température (19) est utilisé pour réguler la température du premier composant (1, 2), un second milieu de régulation de température (20) étant utilisé pour réguler la température du second composant (3, 4), le premier (19) et/ou le second milieu de régulation de température (20) pouvant être un fluide ou un rayonnement électromagnétique, le premier milieu de régulation de température (19) étant amené au premier composant (1, 2) par un premier dispositif de régulation de température (17), le second milieu de régulation de température (20) étant amené au second composant (3, 4) par un second dispositif de régulation de température (18).

8. Procédé selon l'une des revendications précédentes,
**caractérisé par le fait que**
le second dispositif de régulation de température (18) et un dispositif d'application (28) pour l'adhésif (8) sont disposés de manière fixe dans un appareil pour la liaison de deux composants, le second composant (3, 4) étant guidé par un premier dispositif de transport (31) à une position de traitement du second dispositif de régulation de température (18) et/ou par le dispositif d'application (28) pour l'adhésif (8), un second dispositif de transport (32) introduisant le premier composant (1, 2) dans le second composant (3, 4) ou l'attachant au second composant (3, 4).

9. Procédé selon l'une des revendications précédentes,
**caractérisé par le fait que**
le premier composant (1) est un moyen piqueur (2) et le second composant (3) est un corps de seringue (4), le corps de seringue (4) présentant un récipient de stockage (12) dans lequel un milieu destiné à l'administration est apte à être conservé, le récipient de stockage (12) présentant une ouverture proximale (13) du corps de seringue (4), le corps de seringue (4) présentant une région d'extrémité distale (14), dans laquelle est disposé un canal de moyen piqueur (10), le canal de moyen piqueur (10) correspondant au volume de réception (9) du second composant (3), la région d'extrémité distale (14) présentant une ouverture distale (15) du corps de seringue (4), le corps de seringue (4) présentant une région de transition (16) qui est disposée entre la région d'extrémité distale (14) et le récipient de stockage (12).

10. Procédé selon la revendication 9,
**caractérisé par le fait que**
la seconde surface de contact (7) est régulée en température indirectement, au moins une section d'une surface extérieure (3b, 3c, 4b, 4c) de la région d'extrémité distale (14) étant régulée en température au moyen d'un second dispositif de régulation de température (18), où la seconde surface de contact (7) est régulée en température au moyen d'une diffusion de chaleur de la surface extérieure (3b, 3c, 4b, 4c) de la région d'extrémité distale (14) à la seconde surface de contact (7) ou de la seconde surface de contact (7) à la surface extérieure (3b, 3c, 4b, 4c) de la région d'extrémité distale (14).

11. Procédé selon la revendication 9,
**caractérisé par le fait que**
la seconde surface de contact (7) est régulée en température directement, un second milieu de régulation de température (20) étant introduit dans le canal de moyen piqueur (10) de la région d'extrémité distale (4) par un second dispositif de régulation de température (18), le dispositif de régulation de température (18) étant placé à une ouverture distale (15) du canal de moyen piqueur (10), le second milieu de régulation de température (20) étant retiré du corps de seringue (4) par un dispositif de décharge (21), le dispositif de décharge (21) étant disposé dans la région de transition (16), le dispositif de décharge (21) comprenant un dispositif de scellement étanche (22), lequel est disposé de manière étanche sur une paroi intérieure (23) de la région de transition (16), de telle sorte qu'aucun milieu de régulation de température (20) ne pénètre dans le récipient de stockage (12).
